(19) Europäisches Patentamt
European Patent Office
Office européen des brevets

(11) **EP 3 704 466 B1**

(12) **EUROPEAN PATENT SPECIFICATION**

(45) Date of publication and mention
of the grant of the patent:
**11.10.2023 Bulletin 2023/41**

(21) Application number: **18874086.4**

(22) Date of filing: **31.10.2018**

(51) International Patent Classification (IPC):
**G01N 1/40** *(2006.01)*        **G01N 33/48** *(2006.01)*
**G01N 33/49** *(2006.01)*

(52) Cooperative Patent Classification (CPC):
**G01N 33/50; B01L 3/5023; G01N 33/49;**
B01L 2300/0636; B01L 2300/126

(86) International application number:
**PCT/US2018/058440**

(87) International publication number:
**WO 2019/089746 (09.05.2019 Gazette 2019/19)**

(54) **ANALYTE DETECTION METHOD**

ANALYTNACHWEISVERFAHREN

PROCÉDÉ DE DÉTECTION D'ANALYTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.11.2017 US 201762580277 P
10.01.2018 US 201862615576 P**

(43) Date of publication of application:
**09.09.2020 Bulletin 2020/37**

(73) Proprietor: **The Board of Trustees of the Leland
Stanford
Junior University
Stanford, CA 94305-2038 (US)**

(72) Inventors:
• **PALTZ, Gary**
  **Redwood City, CA 94061 (US)**
• **WU, Manhong**
  **Los Gatos, CA 95033 (US)**

(74) Representative: **FRKelly
27 Clyde Road
Dublin D04 F838 (IE)**

(56) References cited:
WO-A1-2011/076859      WO-A1-2016/191738
WO-A1-2017/165630      CA-A1- 2 766 635
US-A1- 2005 136 548    US-A1- 2010 240 079
US-A1- 2017 023 446    US-B1- 6 503 198

• "Recommended Procedure for the Collection of
  Blood Lead by Specimens by Finger Stick",
  Wisconsin State Laboratory of Hygiene, April
  2008 (2008-04), pages 1-5, XP055688994,
  Retrieved from the Internet:
  URL:http://www.slh.wisc.edu/wp-content/upl
  oads/2013/11/Clin-Metals-long_cap.pdf
  [retrieved on 2018-12-19]
• Isopropyl alcohol, 29 October 2017 (2017-10-29),
  page 4, XP55613170, Retrieved from the Internet:
  URL:https://en.wikipedia.org/w/index.php?t
  itle=Isopropyl_alcohol&oldid=807717196
  [retrieved on 2018-12-19]

## Description

### CROSS-REFERENCE

This application claims the benefit of U.S. Provisional Application No. 62/580277, filed on November 1, 2017, and U.S. Provisional Application No. 62/615,576, filed on January 10,2018.

### FIELD OF THE INVENTION

This invention relates to diagnostic measurement of drug or metabolite levels.

### BACKGROUND OF THE INVENTION

For many areas of medicine, the results obtained from diagnostic tests are important for proper diagnosis and treatment. However, existing systems and methods for clinical testing suffer from a number of drawbacks. Presently, current methods are unable to accurately test low amounts of blood. Furthermore, a number of the current methods of testing urine can be easily interfered with, thereby compromising the accuracy of the test results.

The DBS method has been the method of choice for diagnostic tests performed in neonates since 1963 because phlebotomy is not required, the DBS is easily stored, and the DBS can be mailed to an analytical site. More recently, the DBS has been used to monitor the levels of drugs with a narrow therapeutic index, analyze transcriptomic changes, and measure biomarkers.

To prepare a DBS, a standard volume of blood (usually 40 to 50 microliters) from a pinprick is spotted within pre-printed circles (usually 2-4 per subject) on filter paper. The blood is dried, and the material is material subsequently eluted from the filter paper for analysis.

The vast majority of DBS measurements are for genetic testing; they determine whether a particular metabolite is present or absent, and accurate quantitation of the analyte amount is not required. However, drug and analyte measurements require high accuracy. For a DBS measurement to be accurate, a standard blood volume must be placed within a pre-printed circle. Any inhomogeneity introduces variability, and the analyte must be able to be efficiently deposited and eluted from the filter paper. Moreover, a growing number of genetic diseases are screened for, especially in babies (phenylketonuria, maple syrup urine disease, etc.). Such testing, especially those assessing whether an analyte or drug level is changed, require careful quantitation.

Heath care could be improved if low amounts of a sample (such as blood) can be obtained in a minimally invasive manner and if the analytes in samples could be measured accurately. The present disclosure addresses this need and provides a number of advantages as well.

### SUMMARY OF THE INVENTION

A need exists for more rapid and accurate diagnostic methods that will improve the quality of care. Specifically, there is a considerable need for improved methods for sample collection, preparation and analysis for diagnostic testing. A further need exists for easily accessible sample collection sites, which permit the generation of data that can be relied upon by a health care professional.

The present invention is defined in appended independent claim 1 to which reference should be made. Advantageous features are set out in the appended dependent claims.

Systems and diagnostic methods are further needed that will enable earlier intervention, which will provide a high quality of care for patients with little variability and reduced human error. The systems and methods disclosed herein meet this need and provide related advantages as well.

The present disclosure provides a method of preparing and testing low amounts of sample, such as blood, that it is far more accurate than DBS measurements. The methods described herein utilize solvents that are safe for clinical environments, such as isopropanol. In one embodiment, the method requires only 10 microliter of blood or less, such as 8 microliter. Of importance, isopropanol is a commonly used solvent; it is often used for many different analytical purposes, which include: precipitating DNA or RNA, and as a solvent in many column chromatographic procedures that are used for HPLC, mass spectrometry and other analytic methods. However, the present disclosure demonstrates that it has a novel utility; isopropanol can be used as a stabilizing solvent that can be added very small volumes of biological samples, and this enables these samples to be used for analytic testing for determining the levels of many important drugs and metabolites.

In some aspects, the present disclosure provides a method of detecting an analyte, the method comprising: (a) performing an assay on a test solution comprising a sample and a biocompatible solution; and (b) detecting the presence and/or concentration of the analyte in the sample, wherein the biocompatible solution comprises a stabilizing solvent and water, and wherein the sample was obtained through capillary microsampling (CMS).

In some aspects, the present disclosure provides a method of detecting an analyte, the method comprising: (a) performing an assay on a test solution comprising a sample and a biocompatible solution; and (b) detecting the presence and/or concentration of the analyte in the sample, wherein the biocompatible solution comprises a stabilizing solvent and water, and wherein the sample is in the amount of approximately 1-50μL.

In some aspects, the present disclosure provides a method of performing an assay, the method comprising performing an assay on a test solution comprising a sample and a biocompatible solution, wherein the sample is in the amount of 1-50μL, and wherein the error ratio of the assay is <20%.

In some aspects, provided herein is a method for serial monitoring of an analyte in a sample taken from a subject, comprising adding said sample obtained through capillary microsampling (CMS) to a biocompatible solution, wherein the biocompatible solution comprises water and a stabilizing solvent comprising methanol, ethanol, isopropanol or a mixture thereof; wherein the one or more samples are taken at specified time intervals. In some embodiments, the method further comprises comparing one or more of the sample.

In some aspects, the present disclosure provides a method of preparing a sample for detecting an analyte, the method comprising: adding said sample obtained through capillary microsampling (CMS) to a biocompatible solution, wherein the biocompatible solution comprises water and a stabilizing solvent comprising methanol, ethanol, isopropanol or a mixture thereof.

In some embodiments for the methods disclosed herein, the sample is a biological fluid used for diagnostic testing. The biological fluid may be blood, urine, tears, saliva, sweat, sperm or cerebrospinal fluid. In some embodiments, the biological fluid is neonatal blood.

In some embodiments for the methods disclosed herein, the analyte is a drug, nutrient, or metabolite.

In some embodiments for the methods disclosed herein, the stabilizing solvent comprises methanol, ethanol, isopropanol or a mixture thereof. The stabilizing solvent may comprise isopropanol. In some embodiments, the stabilizing solvent is suitable for clinical settings.

In some embodiments for the methods disclosed herein, the biocompatible solution comprises 10-75% V/V of the stabilizing solvent. In some embodiments, the biocompatible solution is substantially free of acetonitrile.

In some embodiments for the methods disclosed herein, the assay is a standard analytic method used for diagnostic testing. The analytic method may be mass spectrometry, chromatography, electrophoresis or enzyme-linked immunosorbent assay (ELISA).

In some embodiments for the methods disclosed herein, approximately 2-50 μL of the sample is obtained from the subject. In some embodiments, the sample is diluted by the biocompatible solution approximately 10-fold.

In some embodiments for the methods disclosed herein, the method further comprises cooling the sample.

In some aspects, provided herein but not part of the invention is a kit comprising: (a) a container configured to obtain a sample from a subject by capillary action; (b) a biocompatible solution; and (c) instructions.

In some embodiments for the kits described herein, the biocompatible solution comprises water and a stabilizing solvent. The stabilizing solvent may comprise methanol, ethanol, isopropanol or a mixture thereof. In some embodiments, the biocompatible solution comprises isopropanol and water.

In some embodiments for the kits described herein, the kit further comprises a device configured to puncture the skin of a subject.

In some aspects, the present disclosure provides a method of detecting an analyte, the method comprising: (a) performing an assay on a test solution comprising a sample and a biocompatible solution; and (b) detecting the presence and/or concentration of the analyte in the sample, wherein the biocompatible solution comprises a stabilizing solvent and water, and wherein the sample was obtained through capillary microsampling (CMS), wherein the sample is in the amount of approximately 1-50μL, and wherein the stabilizing solvent contains isopropanol.

In some embodiments, the sample is a biological fluid used for diagnostic testing. The biological fluid may be blood, urine, tears, saliva, sweat, sperm or cerebrospinal fluid. In some embodiments, the biological fluid is neonatal blood. In some embodiments, the sample is obtained from the subject at a clinical site.

In some embodiments, the analyte is a drug, nutrient, or metabolite. In some embodiments, the metabolite is a marker of a genetic disease or a drug metabolite.

The stabilizing solvent may be suitable for clinical settings.

In some embodiments, the biocompatible solution may comprise 20-30% V/V of the stabilizing solvent.

In some embodiments, the biocompatible solution is substantially free of acetonitrile.

In some embodiments, the assay is a standard analytic method used for diagnostic testing. The analytic method may be mass spectrometry, chromatography, electrophoresis or enzyme-linked immunosorbent assay (ELISA).

Approximately 4-10 μL of sample may be obtained from the subject. In some embodiments, approximately 8 μL of sample is obtained from the subject. In some embodiments, the sample is diluted by the biocompatible solution approximately 10-fold.

The method may further comprise cooling the sample prior to performing an assay.

## BRIEF DESCRIPTION OF THE DRAWINGS

A description of various aspects, features, embodiments, and examples is provided herein with reference to the accompanying drawings, which are briefly described below. The drawings may illustrate one or more aspect(s), feature(s), embodiment(s), and/or example(s) in whole or in part. The drawings are illustrative and are not necessarily drawn to scale.

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

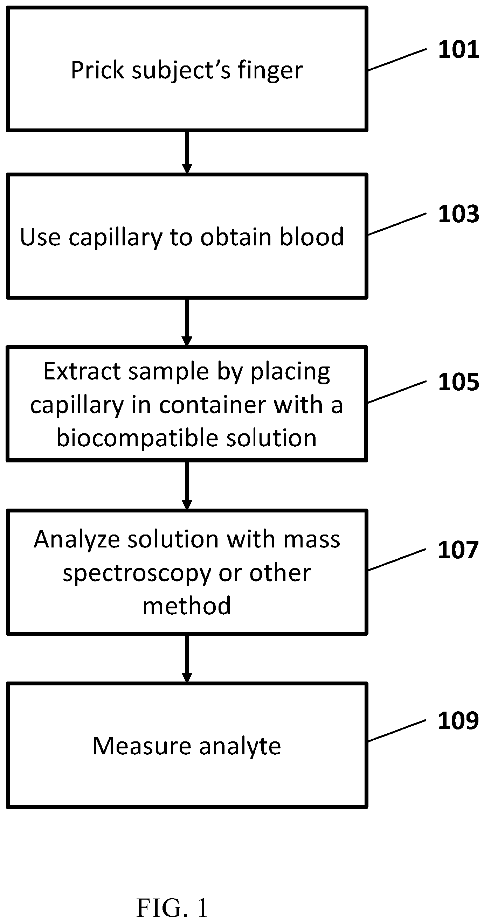

FIG. 1 shows collecting, preparing, processing and analyzing blood from a patient.

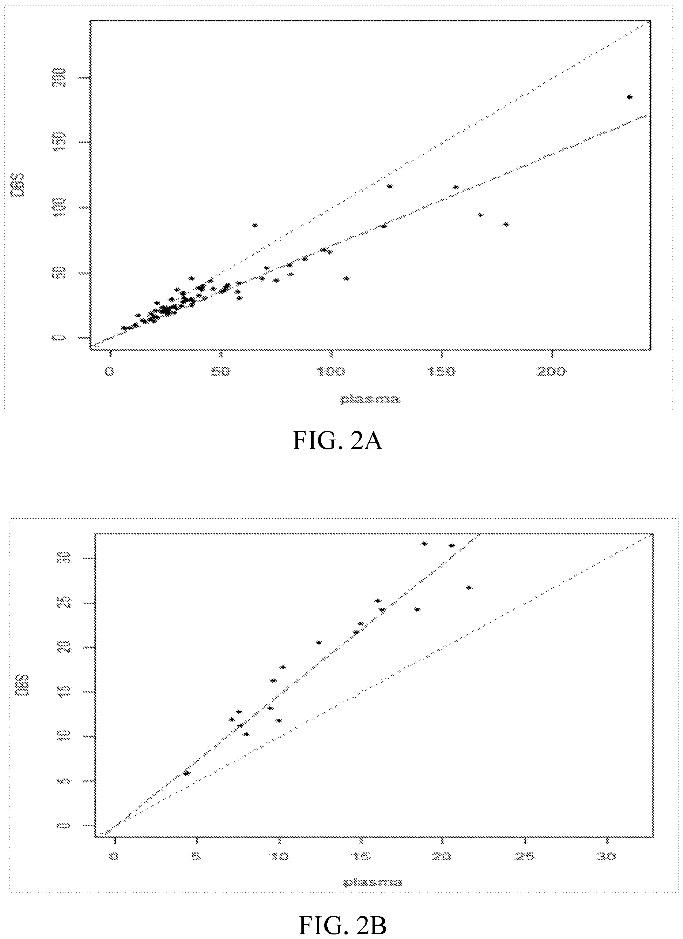

FIG. 2A and FIG. 2B show ondansetron concentrations (ng/ml) [ONDS] that were measured in 20 healthy adults (FIG. 2A) and in 19 newborn baby samples (FIG. 2B) where blood samples were obtained by two different methods. The [ONDS] were measured in plasma samples (x-axis) and from DBS simultaneously obtained from the same subjects (y-axis). The blue dashed lines show the trend lines determined for these samples. The trend lines for the adult (y=0.7063x, left panel) and baby samples (y=1.4726x, right panel) are distinctly different. The theoretical red dotted lines (y=x) indicate the results that would be obtained if identical [ONDS] were measured by the two methods.

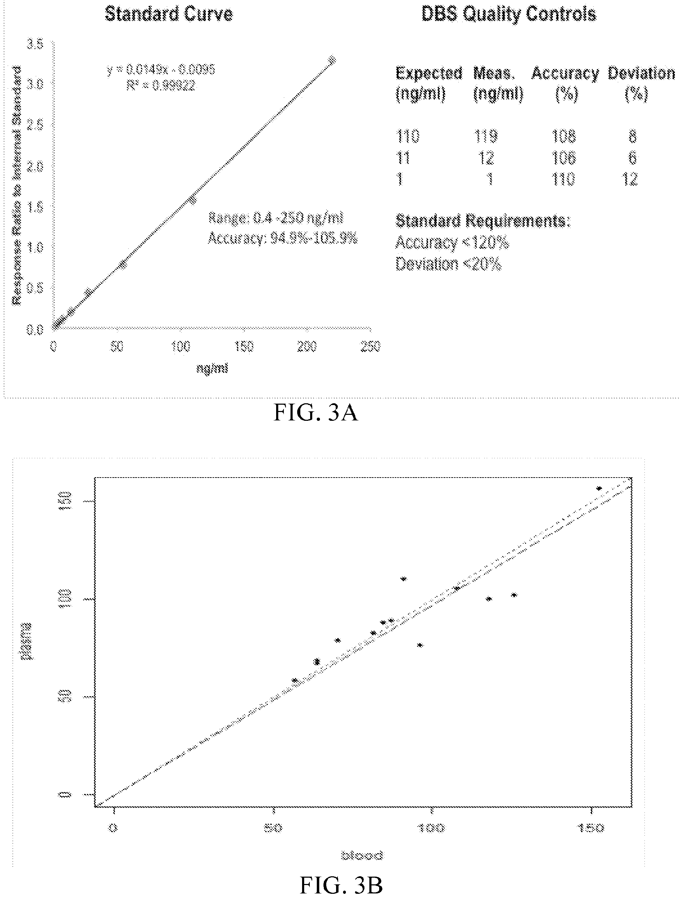

FIG. 3A and FIG. 3B. FIG. 3A depict a standard curve showing the [ONDS] determined using the DBS method. The [ONDS] in the blood used to prepare the DBS ranged from 0. to 250 ng/ml, the $r^2$ for the line was 0.999; and the accuracy was within 20%. The concentrations measured in these samples-which represented the high, middle and low concentrations measured in the subject samples-were all included in the assessment, and the accuracy was within 20%. FIG. 3B: Twelve mice were dosed with ondansetron (10 mg/kg PO), and [ONDS] (ng/ml) were measured in whole blood (x-axis) and in plasma (y-axis) at various times after dosing. The blue dashed trend line (y=0.973x) and the red dotted theoretical line (y=x) lines are shown. 50 microLiter of whole blood was obtained for the plasma analysis, and 20 microLiter of whole blood was obtained for the whole blood analysis.

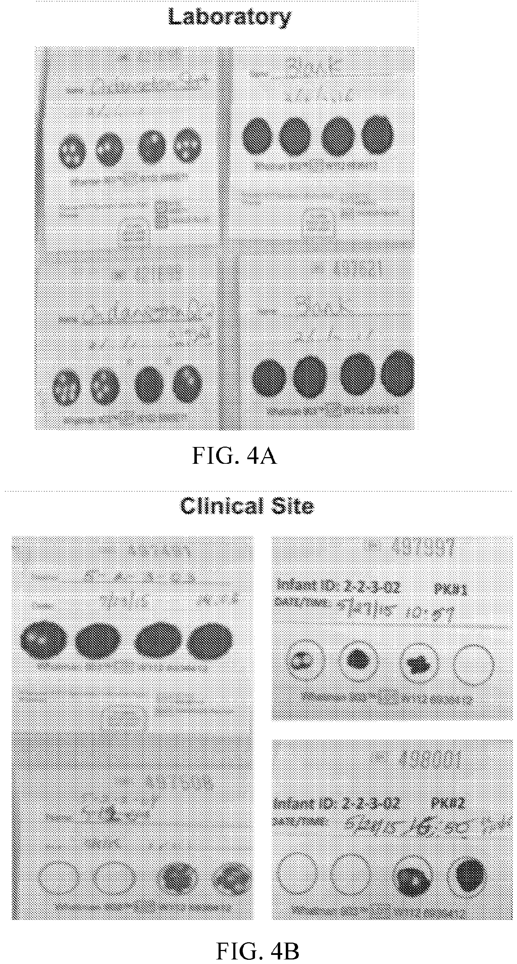

FIG. 4A and FIG. 4B show DBS samples. FIG. 4A shows DBS on filter papers that were carefully spotted in a laboratory setting. Each DBS has 50 microLiter of blood that was spiked with various concentrations of ondansetron. FIG. 4B: Filter papers with DBS obtained from neonates by nurses at the clinical sites are shown. One set of DBS had uniform blood volumes (upper left), but spotted blood volumes on all of the other filters were very inconsistent. Although the same filter papers were used in both settings, there was substantial variability in the spotted blood volume in the clinical setting.

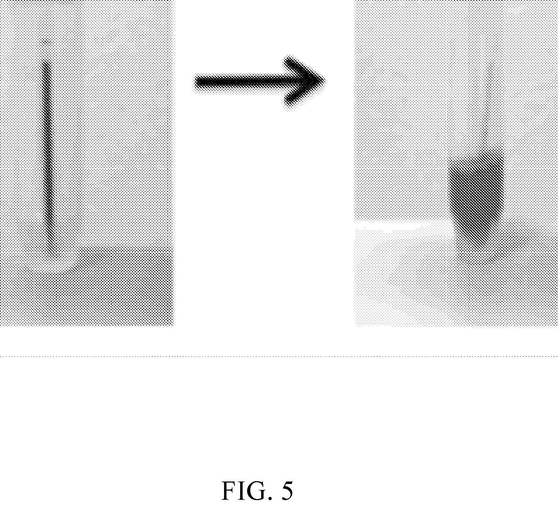

FIG. 5 shows images of a screw cap vial containing 72 microLiter of buffer and an 8 microLiter EDTA-coated capillary tube with mouse blood. The images show the tube before (Left) and after vortexing (Right).

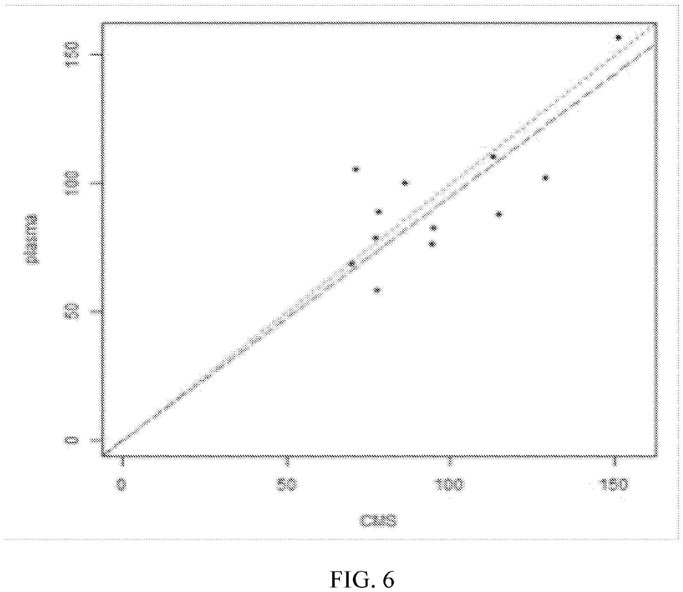

FIG. 6 shows ondansetron concentrations (ng/ml) that were measured in whole blood by capillary microsampling (CMS) (x-axis) or in plasma (y-axis). Blood was obtained from 12 mice at various times after ondansetron (10 mg/kg PO) dosing. The blue dashed trend line (y=0.949x) and red dotted theoretical (y=x) line are shown. Of importance, the CMS method analyzes only 8 microLiter of blood; and the concordance between the theoretical and trend lines indicate that CMS accurately measures the plasma ondansetron concentration.

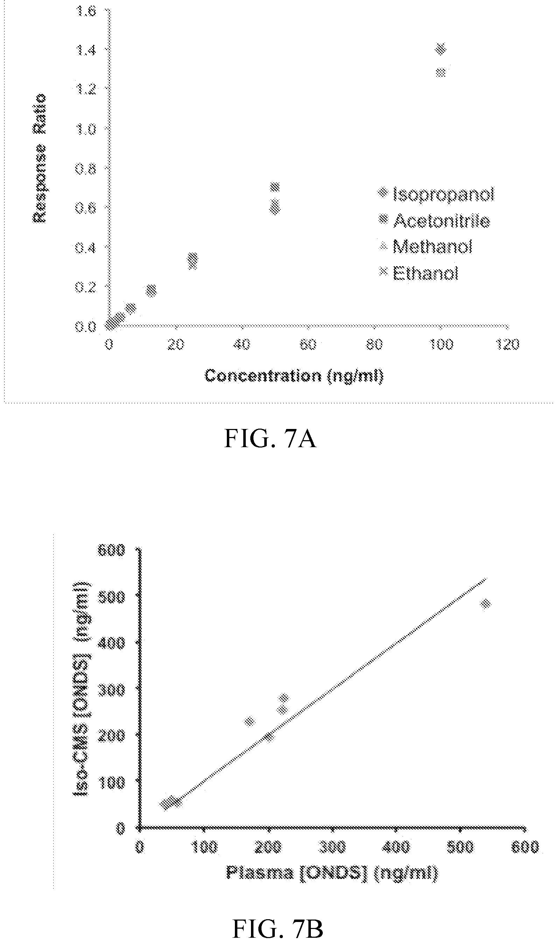

FIG. 7A and FIG. 7B show graphical representations of the response ratio of the [ONDS] in various solutions, and the iso-CMS and plasma measurements of [ONDS] for each sample, respectively. FIG. 7A: The stabilizing solvent does not affect CMS measurements of blood [ONDS]. For these analyses, whole blood was diluted 10-fold into stabilizing solutions that contains 75% water and 25% acetonitrile, isopropanol, methanol or ethanol. As for the generation of a standard curve, the indicated [ONDS] was diluted into the blood/solvent matrix, and the [ONDS] (range: 0.20 -100 ng/ml) were measured by LC/MS analysis. The graph plots the [ONDS] versus the measured response intensity (normalized relative to an internal standard ($d^2$-ondansetron)). The different stabilizing solutions produced identical results. FIG. 7B: Mice were treated with ondansetron (10 or 20 mg/kg PO, n=4 mice per group). Blood was simultaneously obtained 30 minutes later from each mouse by two different methods: (i) 200 microLiter of whole blood for measurement of the plasma [ONDS], or (ii) 8 microLiter for iso-CMS measurement (using isopropanol as the stabilizing solution) of the [ONDS]. The graph compares the iso-CMS and plasma measurements of [ONDS] for each sample. Two features of this graph indicate that the plasma and iso-CMS measurements are highly concordant: 1) the slope of the calculated line (y=0.99x) is virtually identical to the theoretical line of identity (y=x); and 2) the $R^2$ was 0.93, which indicates that the overall deviation of the measured points from the line of identity is very small.

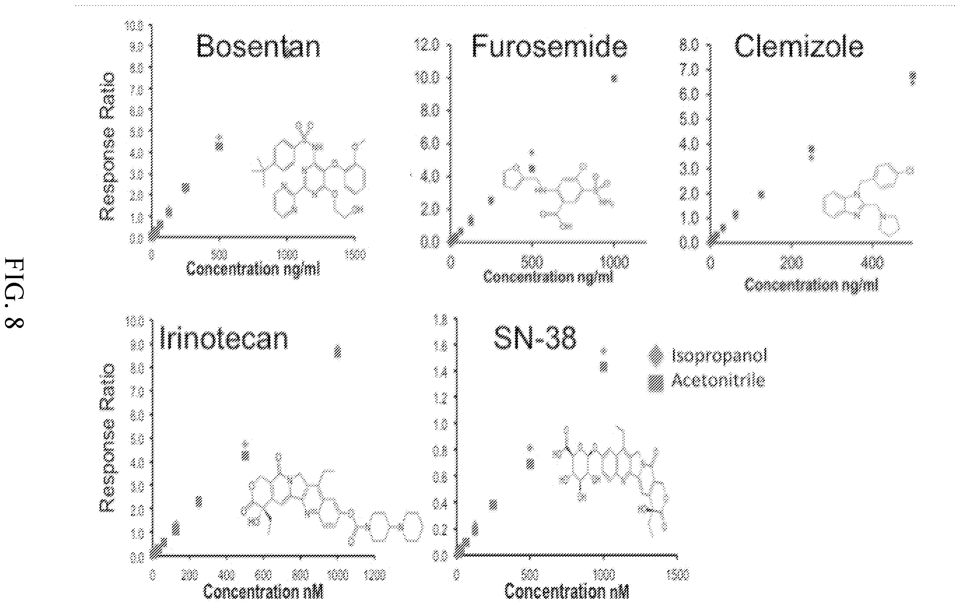

FIG. 8 shows drugs with diverse chemical structures and graphical representations of the response ratio of the drugs using Iso-CMS. The ability of the iso-CMS method to measure the concentrations of the following drugs: bosentan, irinotecan and its metabolite (SN-38), clemizole, and furosemide were tested. For these analyses, whole blood was diluted 10-fold into stabilizing solutions with either 25% acetonitrile or 25% isopropanol and 75% water. As would be done for the generation of a standard curve, each drug was diluted at the indicated concentrations into the blood/solvent matrix, and the drug concentrations were measured by LC/MS analysis using our published methods. The plots depict the measured response intensity (normalized relative to an internal standard with a twice deuterated form of each drug) versus the drug concentration for each tested drug. For each tested drug, the isopropanol stabilizing solution produced results that were identical to that of the acetonitrile stabilizing solution across the range of drug concentrations tested.

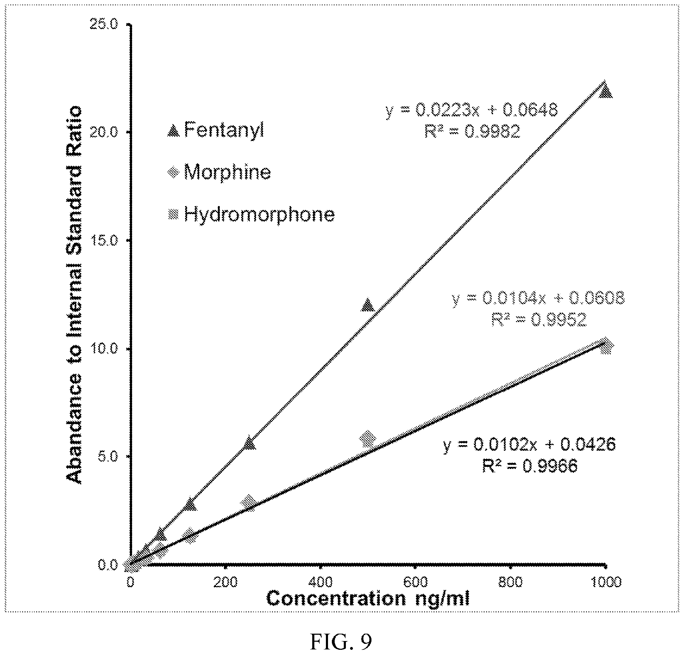

**FIG. 9** shows calibration curves for fentanyl, morphine, and hydromorphon, which were tested at concentrations ranging from 0.5 to 1000 ng/ml. The equation for the trendline and R squared values (all >0.99) are given in the figure.

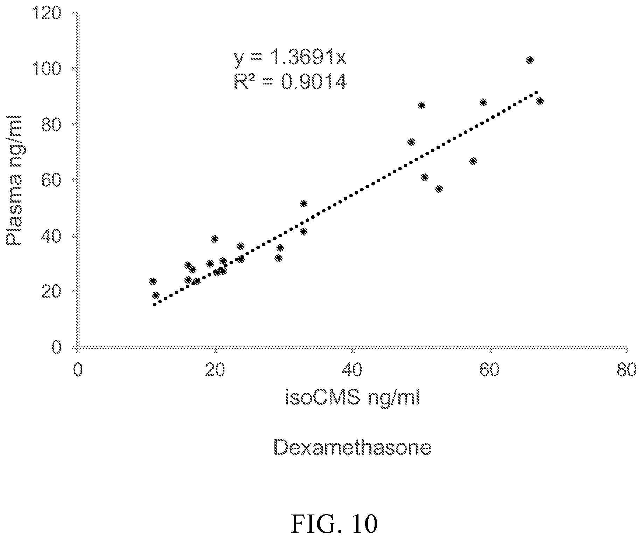

**FIG. 10** shows the dexamethasone concentrations (ng/ml) determined by iso-CMS that correspond with those measured in plasma samples. Plasma and iso-CMS samples were simultaneously obtained from 15 subjects, and the dexamethasone concentrations in each sample were measured by LCMS analysis. Each data point shows the measured dexamethasone concentration determined by analysis of the iso-CMS and plasma sample for each subject. The Pearson correlation and Spearman rank correlation assessing the concordance between the iso-CMS and plasma measurements were 0.95 and 0.93, respectively.

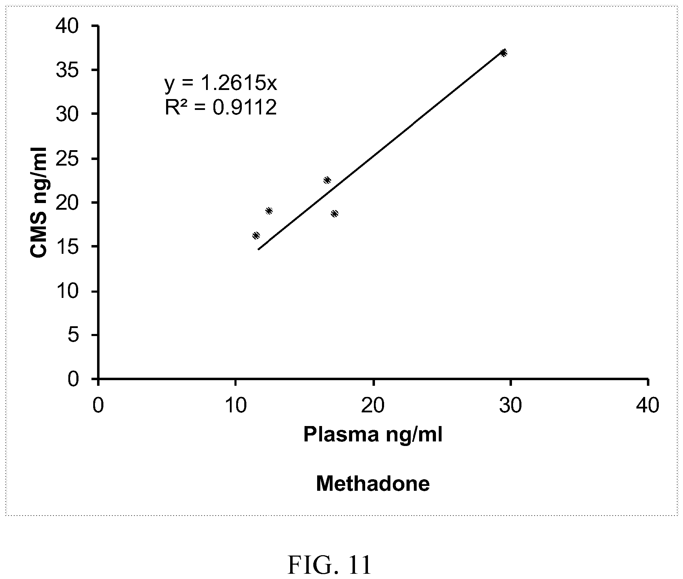

**FIG. 11** shows the methadone concentrations (ng/ml) determined by iso-CMS that correspond with those measured in plasma samples. Plasma and iso-CMS samples were simultaneously obtained from 7 subjects, and the methadone concentrations in each sample were measured by LCMS analysis. Each data point shows the measured methadone concentration determined by analysis of the iso-CMS and plasma sample for each subject. The $R^2$ assessing the concordance between the iso-CMS and plasma measurements was 0.91.

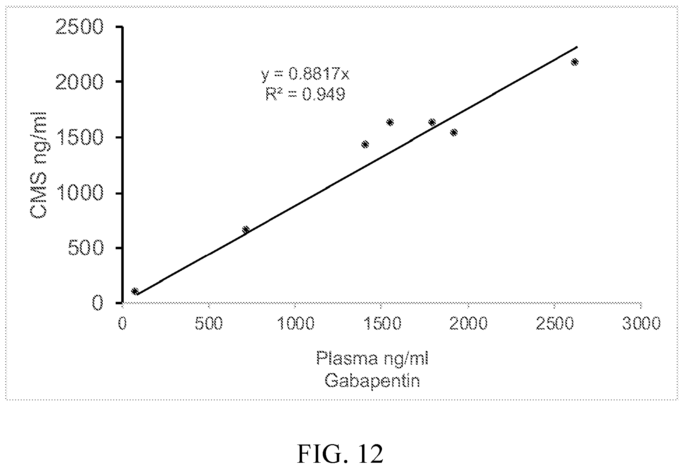

**FIG. 12** shows the gabapentin concentrations (ng/ml) determined by iso-CMS that correspond with those measured in plasma samples. Plasma and iso-CMS samples were simultaneously obtained from 8 subjects, and the gabapentin concentrations in each sample were measured by LCMS analysis. Each data point shows the measured gabapentin concentration determined by analysis of the iso-CMS and plasma sample for each subject. The $R^2$ assessing the concordance between the iso-CMS and plasma measurements was 0.95.

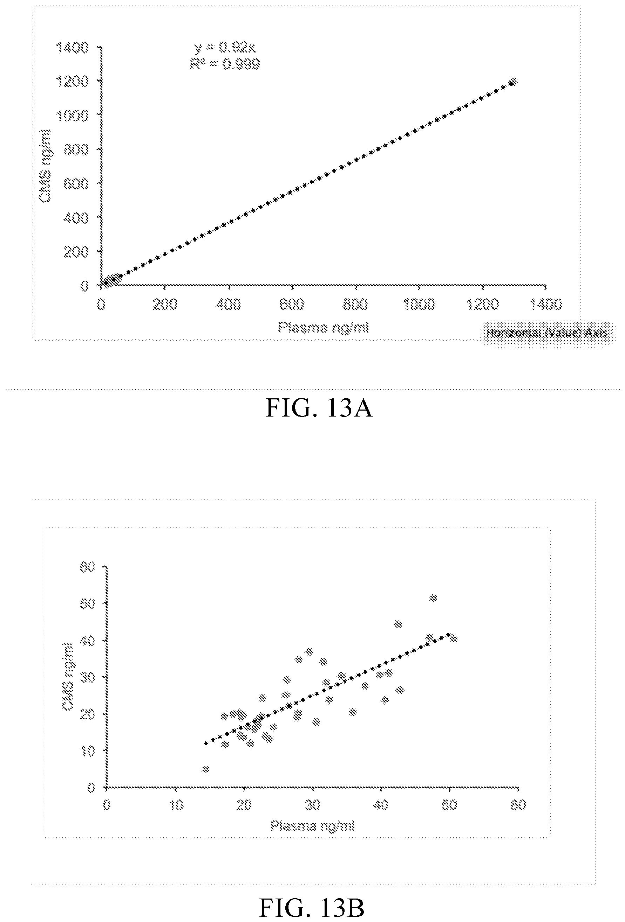

**FIG. 13A and FIG. 13B** show the ondansetron concentrations (ng/ml) determined by iso-CMS that correspond with those measured in plasma samples. **FIG. 13A:** The ondansetron concentrations in 44 simultaneously obtained plasma and iso-CMS samples obtained from surgical paients were measured by LCMS analysis. Each data point shows the measured ondansetron concentration determined by analysis of the iso-CMS and plasma sample for each subject. The $R^2$ assessing the concordance between the iso-CMS and plasma measurements was 0.999. **FIG. 13B**: A region in **FIG. 13A** is expanded to better show the correspondence between the plasma and CMS measurements for samples with ondansetron concentrations between 15 and 60 ng/ml.

## DETAILED DESCRIPTION

Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details or with other methods. Unless stated otherwise, the present invention is not limited by the illustrated ordering of acts or events, as some acts may occur in different orders and/or concurrently with other acts or events. Furthermore, not all illustrated acts or events are required to implement a methodology in accordance with the present invention. The concentration of various components in the disclosed compositions is exemplary and not meant to be limited to the recited concentration per se.

The section headings used herein are for organizational purposes only and are not to be construed as limiting the subject matter described.

This disclosure describes a method for the accurate measurement of molecules in a small sample of biological fluid. These molecules can be metabolites, drugs, components of blood, or other molecules. The measurement could be to diagnose a genetic disease by the measurement of a physiological marker or to determine the amount of drug *in vivo*.

### Definitions

Unless defined otherwise, all technical and scientific terms used herein have the same meaning as is commonly understood by one of skill in the art to which this invention belongs.

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

As used herein, the term "subject" or "individual" includes mammals. Non-limiting examples of mammals include humans and animals, such as mice, including transgenic and non-transgenic mice. The methods described herein can be useful in both human therapeutics, preclinical, and veterinary applications. In some embodiments, the subject is an animal, and in some embodiments, the subject is human. Other mammals include, and are not limited to, apes, chimpanzees, orangutans, monkeys; domesticated animals (pets) such as dogs, cats, guinea pigs, hamsters, mice, rats, rabbits, and ferrets; domesticated farm animals such as cows, buffalo, bison, horses, donkey, swine, sheep, and goats; or exotic animals typically found in zoos, such as bear, lions, tigers, panthers, elephants, hippopotamus, rhinoceros, giraffes, antelopes, sloth, gazelles, zebras, wildebeests, prairie dogs, koala bears, kangaroo, pandas, giant pandas, hyena, seals, sea lions, and elephant seals.

The term "substantially free", as used herein, refers to compositions that have less than about 10%, less than about 5%, less than about 1%, less than about 0.5%, less than 0.1% or even less of a specified component. For example a composition that is substantially free of acetonitrile may have less than about 10% of acetonitrile.

It will be understood that a word appearing herein in the singular encompasses its plural counterpart, and a word appearing herein in the plural encompasses its singular counterpart, unless implicitly or explicitly understood or stated otherwise. Further, it will be understood that for any given component described herein, any of the possible candidates or alternatives listed for that component, may generally be used individually or in any combination with one another, unless implicitly or explicitly understood or stated otherwise. Additionally, it will be understood that any list of such candidates or alternatives, is merely illustrative, not limiting, unless implicitly or explicitly understood or stated otherwise. Still further, it will be understood that any figure or number or amount presented herein is approximate, and that any numerical range includes the minimum number and the maximum number defining the range, whether the word "inclusive" or the like is employed or not, unless implicitly or explicitly understood or stated otherwise. Generally, the term "approximately" or "about" or the symbol "~" in reference to a figure or number or amount includes numbers that fall within a range of $\pm 5\%$ of same, unless implicitly or explicitly understood or stated otherwise. Yet further, it will be understood that any heading employed is by way of convenience, not by way of limitation. Additionally, it will be understood that any permissive, open, or open-ended language encompasses any relatively permissive to restrictive language, less open to closed language, or less open-ended to closed-ended language, respectively, unless implicitly or explicitly understood or stated otherwise.

As used herein, unless otherwise indicated, some inventive embodiments herein contemplate numerical ranges. A variety of aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When ranges are present, the ranges include the range endpoints.

**Challenges with drug testing**

The opioid abuse epidemic has had a major impact on America over the last 10 years. More people are misusing prescription opioids than all other illicit drugs combined. Beginning in 2006, there was a markedly increased number of doctor visits to obtain opioids (32 million per year); and a dramatic shift toward prescribing high potency opioids (such as hydrocodone, and oxycodone) whose 'street value' now far exceeded that of heroin. Because of widespread opiate abuse, laboratory testing for opiate drugs has been integrated into the clinical care of chronic opiate users, as well as for mandated surveillance that is performed in federal and other workplaces.

Although Urine drug testing for opiates (UDTO) is a commonly used surveillance method, it has substantial problems, which could be addressed by the methods, systems, and kits disclosed herein, such as with an iso-CMS-based opiate analysis method (iCOAM). Since urine is collected in private, there are many ways to dilute a sample or to substitute a clean urine specimen. With UDTO, a urine sample can be altered by adding agents that interfere with immunoassays used for opiate detection, which include household products (such as bleach, vinegar, and detergent) or over the counter medications [such as eye drops, NaCl, glutaraldehyde ("Clean-X"), potassium nitrate ('Klear")]. The use of an iCOAM could alleviate these problems since a blood sample is directly obtained via a finger stick from a subject that has properly identified him/herself, and an authorized agent places it into the stabilizing buffer, which eliminates the chance for substitution or alteration.

**Challenges with the Dried Blood Spot (DBS) method**

As shown in FIG. 2A and FIG. 2B, the concentrations of a drug (ondansetron or [ONDS]) measured in plasma samples substantially differed from those measured using DBS. Of importance, the slopes of the trend lines comparing the plasma and DBS measurements generated from healthy adults and from babies were quite different (0.71 vs 1.47) (FIG. 2A and FIG. 2B, respectively). The [ONDS] measured in DBS obtained from neonates was less than that measured in plasma, while this relationship was reversed in adults. A correction factor for converting the [ONDS] measured in DBS to that in plasma, which would work for both adults and babies, could not be found. This discrepancy indicated that there was a systematic problem with the DBS measurements of ondansetron concentration.

Notably, there were inconsistencies in the amount of blood spotted on the filter paper. For DBS measurements to be accurate, a constant blood volume must be spotted within the indicated area of the filter paper. When the DBS is obtained at clinical sites, there is substantial variation in the amount of blood spotted within the indicated area. Inconsistency in the volume of blood spotted on filter paper causes discrepancies in results of tests performed using DBS. This variation significantly alters DBS drug concentration measurements, which are calculated using a standard curve. In this calculation, it is assumed that the amount of blood within the circle is always constant, and a linear equation derived from a standard curve of test samples is used to determine the amount or the change in amount of a drug or metabolite.

The above discrepancies in blood volume are particularly problematic when analyzing neonates. In this case, there is a systemic bias toward placing a smaller amount of blood on the filter paper. In contrast, if a known volume of blood is used for the DBS, the associated variation is eliminated and the drug or metabolite measurements are accurate.

To demonstrate an example of this phenomenon, a series of experiments to investigate the basis for the inconsistencies in the DBS results was performed. 50 microliters of blood was spiked with various concentrations of ondansetron, and carefully spotted onto filter paper in the lab. A reliable standard curve was generated across the range of [ONDS] assayed, and these [ONDS] were similar to those measured in the subjects (**FIG. 3A and FIG. 3B**). These results indicate that [ONDS] could be accurately measured if DBS were carefully prepared in a lab setting. It was also possible that the discrepancy in the clinical sample results could be due to ondansetron partitioning into red blood cells, which are present in blood, but not in plasma. The

[ONDS] in simultaneously prepared whole blood and plasma samples, which were obtained from mice at various times after ondansetron administration, were measured. Since the plasma and whole blood measurements were entirely concordant (**FIG. 3A and FIG. 3B**), differential partitioning of ondansetron could not be responsible for the discrepant results. The discrepancies resulted from inconsistencies in the amount of blood that was spotted on the filter paper were investigated. For DBS measurements to be accurate, a constant blood volume must be spotted within the indicated area of the filter paper. When the blood was carefully spotted in the lab, the DBS had a constant amount of blood. However, the DBS obtained from the clinical sites was examined, there was substantial variation in the amount of blood that was spotted within the indicated area on the filter paper (FIG. 4A and FIG. 4B). Examination of a larger number of clinical samples confirmed that there was substantial variation in the amount of blood spotted on the filter papers.

The discrepant results were caused by inconsistency in the volume of neonatal blood that was spotted on the filter paper at the clinical site. Our results also indicate that when neonates are analyzed, there is a systematic bias toward placing a smaller amount of blood on the filter paper. This explains why the slope of the trend line for the [ONDS] measurements in neonates (**FIG. 2A and FIG. 2B**) was dramatically below that of the adult samples. A larger blood volume was placed on the filter papers when blood was obtained from the adults. This variation will significantly alter the results of drug concentration measurements obtained using DBS. The lab results indicate that if the correct volume of blood is spotted, DBS measurements can produce excellent results; but this can be difficult to achieve when blood is obtained from neonates in a clinical setting.

Current methods for sampling small volumes of blood are not sufficient for accurate analysis and measurement of drugs and metabolites in blood. In some aspects, the methods and systems of the present disclosure address the problem of inconsistent sampling size of low amounts of sample for accurate testing of analytes.

**Capillary Microsampling (CMS) can improve drug measurements**

Studies, which measure the concentrations of different drugs in rodent blood, have shown that the results obtained using CMS methods are quite accurate and reliable. A typical mouse weighs between 20 and 25 grams, and has less than 1.5 mL of total blood volume. Sampling procedures that only require a small amount of blood are necessary for studies where mouse survival is preferred, and CMS measurements use blood volumes that are <10 microLiter. CMS has enabled serial measurement of drug concentrations in rodents for pharmacokinetic and toxicokinetic studies. Moreover, it has been shown in over 50 mouse CMS studies that if the diluted sample is properly vortexed, the sample can be frozen for later analysis. In light of these positive outcomes, the results of CMS studies have been routinely accepted by regulatory authorities.

As a proof of concept, CMS studies in a mouse model were performed. The mouse CMS studies use 8 microLiter glass EDTA-coated capillary tubes, which are obtained from a commercial supplier. After 8 microLiter of mouse blood is collected after a pinprick is made, the capillary tube is placed into a screw cap vial (1.10 ml, V-bottom), with 72 microLiter of an extraction buffer (comprising 25% V/V acetonitrile in water) pre-added. The capped vial is shaken and vortexed vigorously until the blood in the capillary is dissolved in the extraction buffer (**FIG. 5**). The vials are kept on wet ice for 20 minutes, before they are stored in a -80 °C freezer. If needed, the samples can be sent elsewhere for analysis. The study demonstrated that CMS (8 microLiter of blood collected) could be used for serial [ONDS] measurements in mice, and that the CMS results were completely concordant with the [ONDS] measurements from simultaneously obtained mouse plasma (100 microLiter of blood collected per sample) (**FIG. 6**). Moreover, our results indicate that CMS measurements of [ONDS] are significantly more reliable than measurements from DBS, which require at least 10-fold more blood. Thus CMS is suitable for sampling of a small blood volume for the accurate measurement and analysis of drugs and metabolites. Although CMS with acetonitrile showed to be a reliable, the present disclosure provides a new and improved method for CMS analysis.

## A new and improved method for CMS analysis

In some aspects, the present disclosure provides a method of preparing a sample for detecting an analyte, the method comprising: adding said sample obtained through capillary microsampling (CMS) to a biocompatible solution, wherein the biocompatible solution comprises water and a stabilizing solvent comprising methanol, ethanol, isopropanol or a mixture thereof.

The extraction buffer in the CMS experiments described in the previous section contained 25% acetonitrile as a solvent, which creates significant safety issues in a clinical setting. Acetonitrile is metabolized by cytochrome P450 molecules in the liver to produce hydrogen cyanide and formaldehyde, which are both toxic molecules. Toxic effects typically present 2 to 13 hours after exposure, as cyanide accumulates in the body. Toxic effects can be induced by inhaling air containing as little as 100 ppm acetonitrile. Notably, acetonitrile was banned from cosmetics by the European Union in 2000. Therefore, a less toxic extraction buffer is required to enable the clinical personnel obtaining and processing the samples to safely process each sample, and possibly many samples, for analysis and measurement of drugs, metabolites, and other components.

The present disclosure provides a biocompatible solution (or an "extraction buffer") with a solvent with lower toxicity than acetonitrile. In some embodiments, the extraction buffer of the present disclosure is used in clinical settings, such as at a pharmacy or drug store. The extraction may be considered safe to use. Since the extraction buffer has a lower toxicity than acetonitrile, personnel at the clinical site may obtain the sample from the subject and subsequently extract the sample into the extraction buffer at the clinical site. The extracted sample in extraction buffer may be tested on-site for analysis.

The extraction buffer as described herein comprises water and a stabilizing solvent, such as a polar protic solvent. In some embodiments, the stabilizing solvent is isopropanol, methanol, ethanol or a mixture thereof. In one embodiment, the stabilizing solvent is isopropanol. Isopropanol, also known as "rubbing alcohol," may be used given its nontoxic properties. 25% of isopropanol in water can be used in clinical settings. In one embodiment, the extraction buffer comprises water and the stabilizing solution in a ratio of 75/25 (V/V). In some embodiments, the extraction buffer is water and isopropanol at a ratio of 75/25 (V/V), herein referred as "iso-CMS." In some embodiments, the stabilizing solvent is not a polar aprotic solvent.

**FIG. 1** shows an illustrative example of a method of detecting an analyte. The methods and systems described herein for the detection of an analyte generally proceed as follows. A sample is obtained from a subject (e.g., **101**). The sample may be obtained at a clinical site, such as a doctor's office, a pharmacy, a clinic, a drug store, an urgent care center, or the like. The individual collecting the sample from the subject is able to collect the sample in an accurate manner. The individual may use a capillary or a like device to obtain a small volume of a sample, such as biological fluid, from the subject (e.g., **103**). In some embodiments, 1-50 $\mu$L, such as approximately 8 $\mu$L, of sample is obtained. The capillary with the sample is then transferred to a container containing a volume of a biocompatible solution, also known as the extraction buffer, wherein the biocompatible solution comprises water and a stabilizing solvent as described herein, such as isopropanol. The sample is mixed with the stabilizing solvent, such that the sample is transferred into the biocompatible solution and is diluted by the biocompatible solution (e.g., **105**). The sample diluted in the biocompatible solution is the diluted sample. The diluted sample may then be processed or analyzed (e.g., **107**) by one or more techniques as described herein, such as mass spectrometry, to detect and/or measure one or more analytes in the solution (e.g., 109). The diluted sample may also be tested at the clinical site. In some embodiments, the sample is a raw sample or an unprocessed sample.

In some aspects, the present disclosure provides a method of detecting an analyte, the method comprising: (a) performing an assay on a test solution comprising a sample and a biocompatible solution; and (b) detecting the presence and/or concentration of the analyte in the sample, wherein the biocompatible solution comprises a stabilizing solvent as defined in claim 1 and water, and wherein the sample was obtained through capillary microsampling (CMS). In some embodiments, the amount of sample obtained through CMS is about 1-50$\mu$L. In some embodiments, the stabilizing solvent comprises a polar protic solvent, such as isopropanol.

In some aspects, the present disclosure provides a method of detecting an analyte, the method comprising:

(a) performing an assay on a test solution comprising a sample and a biocompatible solution; and (b) detecting the presence and/or concentration of the analyte in the sample, wherein the biocompatible solution comprises a stabilizing solvent as defined in claim 1 and water, and wherein the sample is in the amount of approximately 1-50μL.

In some aspects, provided herein is a method for serial monitoring of an analyte in a sample taken from a subject, comprising adding said sample obtained through capillary microsampling (CMS) to a biocompatible solution, wherein the biocompatible solution comprises water and a stabilizing solvent comprising methanol, ethanol, isopropanol or a mixture thereof; wherein the one or more samples are taken at specified time intervals. In some embodiments, the method further comprises comparing one or more of the sample. The samples may be from the same subject or from different subjects. The sample may be compared against a standard or control. The sample may be monitored for changes in the presence or concentration of an analyte.

In some embodiments, it may be desirable to monitor the amount of drug in a subject's blood over time. The samples may be serially monitored. Generally, serial monitoring protocol comprises collecting more than one sample from the patient using the methods described herein at more than one time interval. The time intervals may be regular or non-regular. The samples may be monitored every minute, every hour, every 2 hours, every 6 hours, every 12 hours, every 24 hours, every 2 days, every week, every month, every year, or an interval comparable to one of these intervals.

For example, a patient may be administered a drug over time, and the plasma levels of the drug are monitored to determine whether the drug concentration in the plasma is in the therapeutic window for that drug. Blood samples may be obtained daily or at some other interval using the CMS methods described herein, and these samples are analyzed to determine the plasma concentration of the drug.

Also described herein is a method of determining a disease or disorder, the method comprising: (a) performing an assay on a test solution comprising a sample and a biocompatible solution; and (b) detecting the presence and/or concentration of the analyte in the sample, wherein the biocompatible solution comprises a stabilizing solvent and water, and wherein the sample was obtained through capillary microsampling (CMS). In some embodiments, the amount of sample obtained through CMS is about 1-50μL. In some embodiments, the disease or disorder is a genetic disease.

**Biocompatible solution**

In some embodiments for the methods, system or kit disclosed herein, the biocompatible solution comprises water and a stabilizing solvent. The stabilizing solvent may be a solvent that has a lower toxicity than acetonitrile. In some embodiments, the stabilizing solvent is one or more polar protic solvents. The stabilizing solvent may be an optionally substituted alkyl alcohol. The stabilizing solvent comprises methanol, ethanol, isopropanol or a mixture thereof. In some embodiments, the stabilizing solvent comprises isopropanol. In some embodiments, the stabilizing solvent consists essentially of isopropanol. The stabilizing solvent may be considered safe to use and may be suitable for clinical settings. In some embodiments, the biocompatible solution is used for extracting the sample from a capillary, such as blood, into the solution. In some embodiments, the stabilizing solvent is not a polar aprotic solvent.

In some embodiments, the biocompatible solution comprises 10-80% V/V of the stabilizing solvent, such as isopropanol. For example, the biocompatible solution may comprise approximately 10%, 15%, 20%, 25%, 30%, 35%, 40%, 45%, 50%, 55%, 60%, 65%, 70%, 75% or 80% V/V of the stabilizing solvent. The biocompatible solution may comprise 10-75%, 15-50%, 15-40%, or 20-30% V/V of the stabilizing solvent. In some embodiments, the biocompatible solution comprises 50-80% V/V of the stabilizing solvent. For example, the biocompatible solution comprises 75% V/V of isopropanol. In some embodiments, the biocompatible solution comprises 20-30% V/V of the stabilizing solvent. In some embodiments, the biocompatible solution comprises approximately 25% V/V of the stabilizing solvent. For example, the biocompatible solution comprises 25% V/V of isopropanol.

In some embodiments, the test solution comprises a low amount of sample in approximately 50-1000μL of the biocompatible solution, such as 50, 75, 100, 150, 200, 250, 300, 350, 400, 450, 500, 550, 600, 650, 700, 750, 800, 900, or 1000μL of the biocompatible solution. The test solution may comprise approximately 50-100μL, 100-200μL, 200-300μE, 300-400μL, or 400-500μL of biocompatible solution. In some embodiments, the test solution comprises a low amount of sample in approximately 50-100μL of biocompatible solution, such as approximately 72μL of biocompatible solution.

In some embodiments, the biocompatible solution comprises 10-500μL of the stabilizing solvent, such as isopropanol. For example, the biocompatible solution may comprise approximately 10, 20, 30, 40, 50, 60, 70, 80, or 90μL of the stabilizing solvent. The biocompatible solution may comprise approximately 100, 110, 120, 130, 140, 150, 160, 170, 180, or 190μL of the stabilizing solvent. The biocompatible solution may comprise approximately 200, 210, 220, 230, 240, 250, 260, 270, 280, or 290μL of the stabilizing solvent. The biocompatible solution may comprise approximately 300, 310, 320, 330, 340, 350, 360, 370, 380, or 390μL of the stabilizing solvent. The biocompatible solution may comprise approximately 400, 410, 420, 430, 440, 450, 460, 470, 480, 490 or 500μL of the stabilizing solvent. In some embodiments, the biocompatible solution comprises 10-100μL, 10-75μL, 10-50μL, 50-150μL, 50-125μL, 50-100μL or 50-90μL of the stabilizing solvent.

In some embodiments, the biocompatible solution is substantially free of a toxic solvent. The biocompatible solution may be free of a toxic solvent. In some embodiments, the biocompatible solution comprises less than 25% of a toxic solvent, such as less than 1%, 2%, 3%, 4%, 5%, 6%, 7%, 8%, 9%, 10%, 11%, 12%, 13%, 14%, 15%, 17%, 18%, 19%, 20%, 21%, 22%, 23%, 24% or 25% of a toxic solvent. In some embodiments, the biocompatible solution comprises less than 5% of a toxic solvent. In some embodiments, the biocompatible solution comprises less than 1% of a toxic solvent.

In some embodiments, the biocompatible solution is substantially free of acetonitrile. The biocompatible solution may be free of acetonitrile. In some embodiments, the biocompatible solution comprises less than 25% of acetonitrile, such as less than 1%, 2%, 3%, 4%, 5%, 6%, 7%, 8%, 9%, 10%, 11%, 12%, 13%, 14%, 15%, 17%, 18%, 19%, 20%, 21%, 22%, 23%, 24% or 25% of acetonitrile. In some embodiments, the biocompatible solution comprises less than 5% of acetonitrile. In some embodiments, the biocompatible solution comprises less than 1% of acetonitrile.

## Measurement of analytes

The methods or systems described herein may be used to measure one or more analytes in a sample. These analytes may be derivatized, preserved, purified, or otherwise subjected to additional processing before, during, or after the performing of any of the methods described herein. Additionally, the analytes may be measured immediately after the collection of the sample, or there may be a delay during which the sample is stored, diluted, derivatized, verified, or otherwise manipulated. In some embodiments, the sample is a raw sample or an unprocessed sample. In some embodiments for the methods or systems disclosed herein, the analyte is a drug, a metabolite, or a nutrient. In some embodiments, anti-cancer agents, antiepileptics, opiates, analgesics, hormonal therapeutic agents, such as steroids, vitamin A, vitamin D and the like are tested for diagnosing a disease or disorder.

**Drugs.** In some embodiments for the methods or systems disclosed herein, the analyte is a drug. The drug may be a cancer drug, an opioid or pharmaceutically active agent. In some embodiments, the analyte is dexamethasone. These drug types include pro-drugs and drug metabolites. In some embodiments, the drug is a combination of drugs.

*Cancer drugs.* Some drugs which can be detected by this method are drugs which are used for the treatment of cancer, known herein as cancer drugs. Cancer is a group of diseasing involving abnormal cell growth, with the potential to invade or spread to other parts of the body, or metastasize. Cancer drugs can be chemotherapeutics, targeted therapeutics, or hormonal therapeutics.

Chemotherapeutics include alkylating agents, antimetabolites, anti-microtubule agents, topoisomerase inhibitors, and cytotoxic antibiotics. Targeted therapeutics include hormone therapies, signal transduction inhibitors, gene expression modulators, apoptosis inducers, angiogenesis inhibitors, immunotherapies, and toxin delivery molecules. Hormonal therapies include drugs which interfere with hormones to stop or slow the growth of hormone-sensitive tumors.

Examples of chemotherapeutic drugs which can be detected by the methods described herein include but are not limited to cyclophosphamide, methotrexate, 5-fluorouracil, doxorubicin, docetaxel, bleomycin, vinblastine, dacarbazine, mustine vincristine, procarbazine, prednisolone, etoposide, cisplatin, epirubicin, capecitabine, folinic acid, and oxaliplatin.

*Opiates.* In some embodiments for the methods disclosed herein, the drug may be an opiate. Iso-CMS based opiate analysis methods (iCOAM) are presented for several opiates of interest, including, but are not limited to, heroin, morphine, codeine, oxycodone and its metabolites (noroxycodone, oxymorphone), hydrocodone and its metabolite (hydromorphone), methadone, buprenorphine fentanyl, hydromorphone, and diamorphine.

*Other drugs.* Other drugs which may be measured using the methods described herein include anti-anxiety, antibiotic, anticoagulant, anticonvulsant, antidepressant, antihistamine, antihypertensive, antihyperlipidemic, antimigrane, antipsychotic, antiviral, beta-adrenergic blocking, benzodiazepine, bronchodilating, calcium channel blocking, corticosteroid, diuretic, anesthetic, neuromuscular blocking, or proton pump inhibiting drugs. The drugs measured may be agonists, inverse agonists, or antagonists.

Some examples of other drugs which can be measured by the methods described herein include but are not limited to warfarin, bosentan, clemazole, gapabentin, and furosemide. Metabolites of drugs which can be measured by the methods described herein include but are not limited to SN-38, oxymorphone, and noroxycodone.

**Metabolites.** In some embodiments for the methods or systems disclosed herein, the analyte is a metabolite. Metabolites as discussed herein are substances formed during metabolism, which includes anabolism and catabolism of a molecule. Metabolites may be intermediate or final products of metabolism. A metabolite may be formed from the metabolism of a drug, a nutrient, or other molecule.

Metabolites located in a biological fluid which can be measured using standard analytic methods including but not limited to mass spectrometry, spectrometry, chromatography, electrophoresis, or ELISA can be measured using the methods described herein.

On some occasions, it is desirable to measure the metabolite of a drug or nutrient rather than the drug or nutrient itself. In some instances it is desirable to measure the metabolite, because the metabolite is toxic, so its levels must be monitored to avoid adverse effects.

In some instances, it is desirable to measure the metabolite, because the metabolite provides some additional benefit, so its levels should be monitored to ensure the benefit is reaped. In some instances, it is desirable to measure the metabolite, because a drug has a very short half-life, but the metabolite has a longer half-life, so the levels of the metabolite may be more indicative of the status of the subject. In some instances, it is desirable to measure the metabolite, because the metabolite is more stable than the drug or nutrient, so the method is more easily or more accurately performed when the metabolite is used. In some instances, it is desirable to measure the metabolite, because the metabolite may provide additional information for research purposes.

**Nutrients.** In some embodiments of the methods or systems disclosed herein, the analyte is a nutrient. If the analyte is a nutrient, it may be a micronutrient or a macronutrient. If the nutrient is a macronutrient, it may be a protein, an amino acid, a carbohydrate, a sterol, or a lipid. If the nutrient is a micronutrient, it may be a vitamin or a mineral.

*Proteins and amino acids.* In some embodiments, the nutrient is a protein or amino acid. Amino acids are organic compounds containing amine and carboxyl functional groups, along with a side chain. Proteins are assembled chains of amino acids.

Amino acids measured by the methods described herein may be aliphatic, aromatic, acidic, basic, hydroxylic, sulfur-containing, amidic, non-essential, or essential.

If the nutrient is an amino acid, it may be phenylalanine, leucine, isoleucine, alloisoleucine, alanine, glycine, proline valine, tryptophan, tyrosine, aspartic acid, glutamic acid, arginine, histidine, lysine, serine, threonine, cysteine, methionine, asparagine, or glutamine.

In some embodiments, amino acids are measured to be used as a diagnostic tool because they are a marker of a genetic disease. For example, phenylalanine is a marker of phenylketonuria (PKU), so phenylalanine may be measured using the methods described herein to diagnose PKU. Additionally, leucine, isoleucine, and alloisoleucine are markers of maple syrup urine disease (MSUD), so they may be measured using the methods described herein to diagnose MSUD. More details regarding these embodiments may be found in the examples section.

*Carbohydrates.* In some embodiments, the methods or systems disclosed herein may be used to measure carbohydrates.

Carbohydrates and their metabolism are essential for health. As such, testing and monitoring of carbohydrates is in the interest of public health. Iso-CMS and the methods described herein can detect carbohydrates in a small volume of sample.

*Sterols.* In some embodiments for the methods disclosed herein, the analyte is a sterol. In some embodiments, the methods or systems disclosed herein are used to measure the sterol cholesterol in the sample. Cholesterol is required by the body to make hormones, vitamin D, and digestive aids. In addition to dietary sources, the body also makes cholesterol. Too much cholesterol can lead to heart disease and other disorders, so monitoring the levels of this molecule is essential.

In other embodiments, the methods or systems disclosed herein are used to measure other sterols, including plant sterols and stanols. When plant sterols and stanols are consumed, they can reduce the circulating cholesterol levels in the subject, thus reducing the subject's risk for heart disease.

*Lipids.* In some embodiments for the methods disclosed herein, the analyte is a lipid. Lipids are fatty acids and their derivative, including triglycerides, diglycerides, monoglycerides, and phospholipids, as well as other sterol-containing metabolites. These molecules are typically insoluble in water, but are soluble in other solvents, including the solvents described in the methods herein. Lipids may be fatty acids, glycerolipids, glycerophospholipids, sphingolipids, sterol lipids, prenol lipids, saccharolipids, or polyketides.

It is of interest to measure and monitor lipids in a subject given the importance of monitoring dietary fat intake and bodily fat status. The methods described herein may be used to measure the lipids using a small sample size.

*Vitamins.* The vitamin may be a vitamin, a provitamin, or a precursor therof. For example, the vitamin may be vitamin A, a B vitamin, vitamin C, vitamin D, or vitamin E. In some embodiments for the methods of the present disclosure, the analyte is vitamin D. Since these vitamins are essential for health, testing and monitoring them to prevent deficiency or excess and diseases associated with these conditions is in the interest of public health.

*Vitamin B.*

Measurement of B-vitamins and their metabolites is possible using several currently available measurement techniques, including mass-spectrometry, chromatographic techniques, and other techniques. The methods provided herein will provide useful for measuring B-vitamins from small samples of biological fluid.

*Vitamin C.*

Vitamin C is a key circulating antioxidant with anti-inflammatory and immune-supporting effects, and a cofactor for important mono and dioxygenase enzymes. An increasing number of preclinical studies in trauma, ischemia/reperfusion, and sepsis models show that vitamin C administered at pharmacological doses attenuates oxidative stress and inflammation, and restores endothelial and organ function. Older studies showed less organ dysfunction when vitamin C was administered in repletion dose (2-3 g intravenous vitamin C/day). Recent small controlled studies using pharmacological doses (6-16g/day) suggest that vitamin C reduces vasopressor support and organ dysfunction, and may even decrease mortality.

Testing and monitoring of vitamin C levels is in the interest of public health. Such testing and monitoring can be performed using small volumes of sample using the methods described herein.

*Vitamin D.* The methods as described herein, such as iso-CMS, are capable of measuring the amount of a nutrient in the blood. This could be a macronutrient or a micronutrient, such as a vitamin or mineral. As an example, vitamin D can be measured by using samples collected using iso-CMS. In some embodiments, the sample can be tested to assess bone health.

Vitamin D is known to play an important role in calcium and bone metabolism, in maternal health during pregnancy and in child development. It has recently been implicated in multiple other medical conditions, which include diabetes, cardiovascular diseases, arthritis, multiple sclerosis and cancer.

Because of their low abundance and low ionization potential, chemical derivatization strategies are used to increase detection sensitivity by introducing ionizable groups onto vitamin D metabolites. A Diels-Alder derivatization reaction is used to react dienophile reagents with the cis-diene group of vitamin D. However, second generation derivatization methods have recently been developed, which now enable a range of vitamin D metabolites to be assayed. These methods allow the assay to be performed, even when the sample size is small, as in iso-CMS.

*Vitamin E.* Vitamin E is a general term describing $\alpha$-, $\beta$-, $\delta$-, and $\gamma$-forms of tocopherol and tocotrienol chemical classes.

Testing and monitoring of vitamin E levels is in the interest of public health. Such testing and monitoring can be performed using small volumes of sample using the methods described herein.

*Minerals.* In some embodiments, the methods described herein may be used to measure the amount of minerals in a sample from a subject. Minerals are consumed as part of the diet of the subject. Minerals are the chemical elements required by living organisms. Appropriate intake levels of each dietary mineral must be sustained to maintain physical health.

The minerals which can be measured by the methods described herein include but are not limited to calcium, phosphorus, potassium, sulfur, sodium chloride, magnesium, iron, zinc, copper, manganese, iodine, selenium, molybdenum, chromium, and fluoride. In each of these cases, the amount of the mineral is essential to health, since either too much or too little can have a deleterious effect on the health of the subject.

### Diagnosis of genetic disease

In some aspects, the present disclosure provides a method of determining a disease or disorder, the method comprising: (a) performing an assay on a test solution comprising a sample and a biocompatible solution; and (b) detecting the presence and/or concentration of the analyte in the sample, wherein the biocompatible solution comprises a stabilizing solvent and water, and wherein the sample is in the amount of approximately 1-50$\mu$L. In some embodiments, the disease or disorder is a genetic disease.

Over 500 diseases are associated with genetic mutations that lead to impaired protein/enzyme production, and these diseases are referred to as inborn errors of metabolism (IEM). Of these, 91 are potentially treatable if they are diagnosed at any early stage. Moreover, 13 of the treatable IEM are disorders affecting amino acid production or catabolism (aminoacidopathies): Phenylketonuria (PKU), Maple Syrup Urine Disease (MSUD), Homocystinuria/Methylene Tetrahydrofolate Reductase (MTHFR) deficiency, Tyrosinemia type II, Citrullinemia type I and type II, Argininosuccinic aciduria, Carbamoyl Phosphate Synthetase I (CPS) deficiency, Argininemia (arginase deficiency), Hyperornithinemia-Hyperammonemia-Homocitrullinuria (HHH) syndrome, N-Acetylglutamate Synthase (NAGS) deficiency, Ornithine Transcarbamylase (OTC) deficiency, and Pyruvate Dehydrogenase (PDH) complex deficiency. The financial and psychological burdens caused by these IEM are reduced if effective newborn screening programs are in place. If a diagnosis is made early, a dietary or other modification can be put in place to prevent the damage caused by the disease.

### Samples

Any of the methods or systems described herein, including iso-CMS, are also useful for measurements of samples, such as blood and other biological fluids. In some embodiments, the sample is a biological fluid used for diagnostic testing. Iso-CMS allows for the non-invasive sampling of blood, tears, sweat, cerebrospinal fluid (CSF), and other bodily fluids for analysis.

In some embodiments, the sample is selected from the group consisting of blood, serum, plasma, nasal swab or nasopharyngeal wash, saliva, urine, tears, gastric fluid, spinal fluid, stool, mucus, sweat, earwax, oil, glandular secretion, cerebral spinal fluid, tissue, semen, and vaginal fluid, throat swab, breath, hair, finger nails, skin, biopsy, placental fluid, amniotic fluid, cord blood, emphatic fluids, cavity fluids, sputum, mucus, puss, micropiota, meconium, breast milk and/or other excretions. In some embodiments, the biological fluid is blood, urine, tears, saliva, sweat, sperm or cerebrospinal fluid. In some embodiments, the sample is blood. In some embodiments, the sample is neonatal blood.

In some embodiments, low amounts of sample, such as blood is obtained from a subject. In some embodiments, the amount of sample obtained from a subject is about or at least about 2µL, 3 µL, 4 µL, 5µL, 6µL, 7µL, 8µL, 9µL, 10µL, 15µL, 20µL, 25µL, 30µL, 40µL, 50µL, 60µL, 70µL, 80µL, 90µL, 100µL, 500µL, 1mL, 2mL, 3 mL, 4 mL, 5mL, 6mL, 7mL, 8mL, 9mL, or 10mL. In some embodiments, approximately 2-100µL of sample is obtained from the subject and/or used for testing. For example, 2, 3, 4, 5, 6, 7, 8, 9, 10, 15, 20, 25, 30, 40, 50, 60, 70, 80, 90 or 100µL of sample is obtained from the subject and/or used for testing. In some embodiments, the amount of sample obtained from the subject is or is approximately 2-25µL, 4-15µL, or 4-10µL and/or is used for testing. In some embodiments, approximately 8 µL of sample is obtained from the subject and/or used for testing. In some embodiments, the amount of sample obtained from a subject is diluted with a biocompatible solution prior to analysis.

In some embodiments, the sample is diluted by the biocompatible solution. In some embodiments, the sample is diluted by the biocompatible solution approximately 3 to 20-fold, for example 3, 4, 5, 6, 7, 8, 9, 10, 11, 12, 13, 14, 15, 16, 17, 18, 19, or 20-fold. The sample may be diluted by the biocompatible solution approximately 5 to 15-fold. In some embodiments, the sample is diluted by the biocompatible solution approximately 10-fold.

In some embodiments for the methods described herein, the methods further comprise cooling the sample. The samples may be cooled to preserve the sample for use at a later time. For example, the sample may be placed on ice, in cool water, or in temperature-controlled unit, such as a fridge or freezer. Cooling the sample may comprise maintaining the sample at a temperature approximately below 0°F. In some embodiments, the sample is cooled prior to performing the assay. The sample may be previously frozen and thawed prior to performing an assay. In some embodiments for the methods described herein, the methods further comprise adding an anticoagulant or a preserving agent.

In practicing any of the methods above or elsewhere herein, alone or in combination, the qualitative and/or quantitative evaluation of the sample may be effected with or without transporting the sample from the site where the sample is collected to a facility capable of testing the sample. In some embodiments, the facility is a clinical site, such as a pharmacy or drug store. Assays

The methods or systems described herein may be combined with one or more of any assay that is a standard analytic method that can be used for diagnostic testing. The set of assays include, but is not limited to mass spectrometry, spectroscopy, chromatography, electrophoresis, or enzyme-linked immunosorbent assays (ELISA).

Mass spectrometry is an analytical technique that analyzes chemical species, and it separates the ions generated from the analytes based on their mass-to-charge ratio. Mass spectrometry can be applied for the analysis of pure samples, as well as complex samples such as the biological fluids described herein.

Spectroscopy is the analysis of the sample on the basis on the interaction between electromagnetic radiation and the sample. Devises used for the spectroscopic analysis of a sample include spectrometers, spectrophotometers, spectrographs, and spectral analysers.

Chromatography is the separation of a mixture by passing it in solution or as a vapor through a medium in which the components move at different rates. In this way, the components of the sample can be separated; which enables them to be identified and quantified.

Chromatography may be combined with mass spectrometry or spectroscopy for the analysis of the sample. Examples of such a combination include LCMS (liquid chromatography / mass spectrometry), and GCMS (gas chromatography / mass spectrometry).

In some aspects, the present disclosure provides a method of performing an assay, the method comprising performing an assay on a test solution comprising a sample and a biocompatible solution, wherein the sample is in the amount of 1-50µL, and wherein the error ratio of the assay is equal to or less than 20%. In some aspects, the present disclosure provides a method of performing an assay, the method comprising performing an assay on a test solution comprising a sample and a biocompatible solution, wherein the sample is obtained by CMS, and wherein the error ratio of the assay is less than 20%.

As used herein, the error ratio (ER) is a normalized version of the deviation of the amount of analyte measured from the methods described herein from the amount of analyte measured from plasma collected from a gold standard method. is calculated as:

$$ER = \frac{|A_{CMS} - A_{Plasma}|}{A_{Plasma}} x100\%$$

Where $A_{CMS}$ is the amount of the analyte measured from the sample obtained by means of the CMS methods described herein, and $A_{Plasma}$ is the amount of the analyte measured from a sample obtained at the same time by means of a gold standard method, where the gold standard method can be plasma obtained by venipuncture. An ER of 0 means that the method is in perfect agreement with the gold standards. A low ER (approaching 0) indicates that the method produces results similar to those of the gold standard, while a high ER indicates that the method produces results dissimilar to those of the gold standard. The range of the ER may be from 0 to 100%.

**Subjects**

In any of the methods or systems described herein, the subject is a human or an animal. The subject may be an infant, a child, an adolescent, an adult, or an elderly subject. The subject may have or be at risk for a genetic disease, chronic pain, cancer, an infectious disease, cardiovascular disease, nutrient deficiency, a metabolic disease, drug addiction, or other ailment. The subject may be pregnant, such as a pregnant female. In some embodiments, the subject is a neonate. In some embodiments, the subject has a nutrient deficiency.

In some embodiments of any of the methods described herein, the subject is a human. For example, the human subject may be an adult, a child, or an infant. In an exemplary embodiment, the human subject is a human adult. The human subject may be a human adult 18 years old or older. The human subject may be between the ages 18 and 90 years old. In a certain embodiment, the human subject is between 40 and 85 years old. In an exemplary embodiment, the human subject is between 65 and 80 years old.

In some embodiments of any of the methods described herein, the subject is an animal. The animal subject may be a domesticated animal, such as a dog or cat. The animal subject may be a mouse or rat. The animal subject may be a mouse or rat at least 7 weeks old. The animal subject may be a mouse or rat between 7 and 15 weeks old. The animal subject may be a mouse or rat between 10 and 11 weeks old.

*Genetic testing patient.* If the subject has a genetic disease or is at risk for a genetic disease, any of the methods described herein may be used to analyze the sample for the presence of a marker of that genetic disease. In two notable embodiments, the subject is at risk for PKU or MSUD. If the subject is at risk for PKU, any of the methods described herein may be used to measure phenylalanine levels, since phenylalanine is a marker for PKU.

If the subject is at risk for MSUD, any of the methods described herein may be used to analyze levels of branched chain amino acids, including leucine, isoleucine, and alloisoleucine, which are markers for MSUD.

If the subject is an infant, the subject may be at risk for a genetic disease, and any of the methods described herein can be used to test the subject for PKU, MSUD, or any other genetic disease as described herein.

*Chronic pain.* If the subject is a chronic pain patient, the patient may have a prescription for one or more opioids or other pain medications. In this embodiment, the sample for the patient may be analyzed for the presence of prescribed or non-prescribed opioids or other pain medications to monitor treatment safety and dependence risk.

*Nutrient deficiency.* If the subject has a nutrient deficiency or is at risk for a nutrient deficiency, any of the methods described herein may be used to analyze the sample for deficiency of that nutrient. In this case, the nutrient may be a macronutrient or a micronutrient. If the nutrient is a macronutrient, it may be a protein, an amino acid, a carbohydrate, or a lipid. If the nutrient is a micronutrient, it may be a vitamin or a mineral. If the nutrient is a vitamin, it may be vitamin A, vitamin B, vitamin C, vitamin D, vitamin E, vitamin K, or any of the precursors or metabolites of these vitamins. If the nutrient is a mineral, it may be calcium, phosphorus, potassium, sulfur, sodium chloride, magnesium, iron, zinc, copper, manganese, iodine, selenium, molybdenum, chromium, or fluoride.

*Infectious disease.* If the subject has or is at risk for an infectious disease, any of the methods described herein may be used to analyze the sample for markers of the infectious disease or for antibiotics, antivirals, antifungals, or antiparasitic drugs given to the subject for the treatment of the infectious disease.

*Cardiovascular disease.* If the subject has or is at risk for cardiovascular disease, any or the methods described herein may be used to analyze the sample for cholesterol. Also in this embodiment, any of the methods described herein may be used to analyze the sample for drugs used in the treatment of cardiovascular disease, including but not limited to statins, inotropic drugs, chronotropic drugs, or other drugs which can treat cardiovascular disease.

*Cancer patients.* Subjects utilizing the methods of the present disclosure include subjects that have been diagnosed as having acute myeloid leukemia, acute myeloid leukemia, cancer in adolescents, adrenocortical carcinoma childhood, AIDS-related cancers (e.g., Lymphoma and Kaposi's Sarcoma), anal cancer, appendix cancer, astrocytomas, atypical teratoid, basal cell carcinoma, bile duct cancer, bladder cancer, bone cancer, brain stem glioma, brain tumor, breast cancer, bronchial tumors, burkitt lymphoma, carcinoid tumor, atypical teratoid, embryonal tumors, germ cell tumor, primary lymphoma, cervical cancer, childhood cancers, chordoma, cardiac tumors, chronic lymphocytic leukemia (CLL), chronic myelogenous leukemia (CML), chronic myleoproliferative disorders colon cancer, colorectal cancer, craniopharyngioma, cutaneous T-cell lymphoma, extrahepatic ductal carcinoma in situ (DCIS), embryonal tumors, CNS cancer, endometrial cancer, ependymoma, esophageal cancer, esthesioneuroblastoma, ewing sarcoma, extracranial germ cell tumor, extragonadal germ cell tumor, eye cancer, fibrous histiocytoma of bone, gall bladder cancer, gastric cancer, gastrointestinal carcinoid tumor, gastrointestinal stromal tumors (GIST), germ cell tumor, gestational trophoblastic tumor, hairy cell leukemia, head and neck cancer, heart cancer, liver cancer, hodgkin lymphoma, hypopharyngeal cancer, intraocular melanoma, islet cell tumors, pancreatic neuroendocrine tumors, kidney cancer, laryngeal cancer, lip and oral cavity cancer, liver cancer, lobular carcinoma in situ (LCIS), lung cancer, lymphoma, metastatic squamous neck cancer with occult primary, midline tract carcinoma, mouth cancer multiple endocrine neoplasia syndromes, multiple myeloma/plasma cell neoplasm, mycosis fungoides, myelodysplastic syndromes, myelodysplastic/myeloproliferative neoplasms, multiple myeloma, merkel cell carcinoma, malignant mesothelioma, malignant fibrous histiocytoma of bone and osteosarcoma, nasal cavity and paranasal sinus cancer, nasopharyngeal cancer, neuroblastoma, non-hodgkin lymphoma, non-small cell lung cancer (NSCLC), oral cancer, lip and oral cavity cancer, oropharyngeal cancer, ovarian cancer, pancreatic cancer, papillomatosis, paraganglioma, paranasal sinus and nasal cavity cancer, parathyroid cancer, penile cancer, pharyngeal cancer, pleuropulmonary blastoma, primary central nervous system (CNS) lymphoma, prostate cancer, rectal cancer, transitional cell cancer, retinoblastoma, rhabdomyosarcoma, salivary gland cancer, skin cancer, stomach (gastric) cancer, small cell lung cancer, small intestine cancer, soft tissue sarcoma, T-Cell lymphoma, testicular cancer, throat cancer, thymoma and thymic carcinoma, thyroid cancer, transitional cell cancer of the renal pelvis and ureter, trophoblastic tumor, unusual cancers of childhood, urethral cancer, uterine sarcoma, vaginal cancer, vulvar cancer, Viral-Induced cancer, leukemia, hematologic malignancy, solid tumor cancer, prostate cancer, castration-resistant prostate cancer, breast cancer, Ewing's sarcoma, bone sarcoma, primary bone sarcoma, T-cell prolymphocyte leukemia, glioma, glioblastoma, hepatocellular carcinoma, liver cancer, or diabetes, related conditions, and combinations thereof. In some embodiments, the subjects treated with a food composition, liquid composition, and/or dry composition of the present disclosure include subjects that have been diagnosed as having a non-cancerous hyperproliferative disorder such as benign hyperplasia of the skin (e.g., psoriasis), restenosis, and prostate (e.g., benign prostatic hypertrophy (BPH). The subject may have a genetic predisposition for cancer.

**Systems**

A system for evaluation of a sample collected from a subject to aid in diagnosis, treatment, or prevention of a disease may be provided in accordance with an additional aspect of the invention. In some aspects, described herein is a system of evaluating a sample collected from a subject, the system comprising (a) a communication unit configured to receive data from a device, wherein the device is configured to process the sample, thereby generating data necessary for a subsequent qualitative and/or quantitative evaluation of said sample, and wherein the device comprises (i) a sample collection unit configured to receive the sample; (ii) a sample preparation unit configured to prepare the biological sample for the subsequent qualitative and/or quantitative evaluation, wherein the sample preparation unit permits the biocompatible solution to be added to the blood sample; and (iii) transmission unit configured to transmit the data to an authorized analytical facility and/or an affiliate thereof; and (b) a processor that processes said data for the subsequent qualitative and/or quantitative evaluation of said blood sample at the authorized analytical facility and/or the affiliate thereof, wherein the biocompatible solution comprises water and a stabilizing solvent. The sample may be collected at a clinical site or a laboratory.

The systems described above or elsewhere herein, alone or in combination may include a device that is configured to receive information relating to said qualitative and/or quantitative evaluation and optionally display said information on said device. The systems described above or elsewhere herein, alone or in combination may include the detection of an analyte, such as a drug, metabolite or nutrient. In some embodiments, the sample is collected by CMS. In some embodiments, the sample is collected at health care provider's office, hospital, clinic, drug store, pharmacy, or medical facility. In some embodiments, the sample is collected at a pharmacy. In some embodiments, the sample is collected at home. In systems above or elsewhere herein, alone or in combination, the qualitative and/or quantitative evaluation may involve detecting the presence or concentration of an analyte.

In some systems above or elsewhere herein, alone or in combination, the biological sample may be collected from a pinprick. In some embodiments, approximately 2-100$\mu$L of sample is obtained from the subject and/or used for testing. In some embodiments, the biological fluid is blood, urine, tears, saliva, sweat, sperm or cerebrospinal fluid.

**Facilities**

**[0139]** [0073] Described herein are facilities for making and/or administering the kits described herein as well as facilities for using the methods described herein. In some aspects, the present description provides a facility comprising a plurality of kits described herein. In certain embodiments, the facility manufactures the kits described herein or components thereof. In one embodiment, the facility is a manufacturing site. In certain embodiments, the facility distributes the kits described herein or components thereof. For example, the facility may be a distribution center. In certain embodiments, the facility provides the kits described herein and components thereof to a patient, health care provider, hospital, clinic, drug store, pharmacy, medical facility and the like. In one embodiment, the facility may sell the kit to a drug store. In another embodiment, the facility may prepare the kit disclosed herein. In another embodiment, the facility may administer the kit to the subject. For example, the facility may be a hospital, clinic, drug store, pharmacy or a medical facility. The facility may be located in or within the vicinity of a hospital, clinic, drug store, pharmacy or a medical facility, such as within 0.1, 0.5, 1, or 5 miles of a hospital, clinic, drug store, pharmacy or a medical facility.

**Kits**

Provided herein are kits. The kits may be used for any of the methods and/or systems disclosed herein. The kits may include containers and solutions described herein, in suitable packaging, and written material that can include instructions for use, listing of side effects, and the like. In some aspects, the present description provides a kit comprising: (a) a container configured to obtain a sample from a subject by capillary action, (b) a biocompatible solution, and (c) instructions. Such kits may also include information, such as scientific literature references, package insert materials, and/or summaries of these and the like, which indicate or establish the activities and/or advantages of the methods described herein, and/or which describe administration, side effects, or other information useful to the health care provider and/or patient. In some embodiments, the container may be configured to extract low amounts of sample from the subject, such as 1-50μL. In some embodiments for the kits described herein, the biocompatible solution comprises water and a stabilizing solvent. The stabilizing solvent may comprise a polar protic solvent. The stabilizing solvent may comprise methanol, ethanol, isopropanol or a mixture thereof. In some embodiments, the biocompatible solution comprises isopropanol and water. In some embodiments the kit further comprises an anticoagulant or the like for sample preservation. In some embodiments the kit further comprises a device configured to puncture the skin of a subject. In some embodiments the kit further comprising a capillary. In some embodiments, the instructions describe performing capillary microsampling (CMS) to draw a sample into a capillary, inserting the capillary into the container with the biocompatible solution, and agitating the container until the sample is transferred to the biocompatible solution.

In some embodiments, the container for the sample and the biocompatible solution are provided in separate containers within the kit. In some embodiments, the container for the sample and the biocompatible solution are provided as one container within the kit. Suitable packaging and additional articles for use (e.g., measuring cup for liquid preparations, foil wrapping to minimize exposure to air, and the like) are known in the art and may be included in the kit. Kits described herein can be provided, marketed and/or promoted to health providers, including physicians, nurses, pharmacists, formulary officials, and the like. Kits may also, in some embodiments, be provided, marketed and/or promoted directly to the consumer.

In some embodiments, a kit described herein can comprise a supply for multiple testing. The kit can comprise instructions directing the use of the supply over a period of multiple days, weeks or months.

**EXAMPLES**

**Example 1: Stabilizing solvent identification for CMS and iso-CMS methods.**

To identify an alternative solvent, the standard curves and detection sensitivities for blood [ONDS] measurements were examined, which were developed under CMS conditions performed as in **FIG. 3A and FIG. 3B.** Whole blood was diluted 10-fold into stabilizing solutions comprising water and one of acetonitrile, isopropanol, methanol or ethanol (25% V/V). the toxicity of each of these solvents is lower than for acetonitrile, i.e., a higher dose is required for toxic effects to present themselves. These were then used to assay blood samples that were spiked with a range of [ONDS]. Assays were performed as described above. All of the tested extraction solvents produced the same results as the acetonitrile control sample (FIG. 7A). Isopropanol was selected for further testing and use as the stabilizing solvent for [ONDS] measurements in clinical settings. Again, these tests and measurements were performed as described above. The study demonstrated that the performing the CMS method using a stabilizing solution comprising isopropanol (25% V/V) in water. (iso-CMS) could accurately determine the [ONDS] in mice after dosing with ondansetron (FIG. 7B). Importantly, the [ONDS] determined by the iso-CMS measurements were identical to those measured by analysis of plasma.

The concentrations of multiple other drugs with diverse chemical structures in samples procured using the iso-CMS method can be accurately measured. Namely, iso-CMS accurately measured the concentrations of bosentan, furosemide, clemizole, irinotecan, and the irinotecan metabolite SN-38. Known amounts of these drugs were added to small volume blood samples which had been collected and diluted into the stabilizing solution used for either the CMS (with acetonitrile) or iso-CMS (isopropanol) method. For these analyses, whole blood was diluted 10-fold into stabilizing solutions with either 25% acetonitrile or 25% isopropanol and 75% water Concentrations of the drugs were then measured using LC-MS analysis. Plots depicting the response ratio, or measured response intensity, with respect to the concentration of drug in the sample were created for each drug. Representative plots depicting typical data can be found in **FIG. 8.** Linear regression analysis of the results of the analysis indicated that the iso-CMS method measured these drugs with linearity and sensitivity similar to that of the method using acetonitrile. In fact, for each tested drug, the isopropanol stabilizing solution produced results that were identical to that of the acetonitrile stabilizing solution across the range of drug concentrations tested. Thus, the volume of the blood sample is sufficiently consistent in this method, and the drugs are sufficiently soluble; accurate measurements were obtained when isopropanol was used (and acetonitrile was omitted) from the stabilizing solution. In light of the above mentioned results, similar performance of the iso-CMS method is expected when methanol, ethanol, or a mixture thereof is used in the composition of the stabilizing solution.

**Example 2: Detecting blood markers with iso-CMS**

In this example, blood markers for a genetic disease were accurately measured by the iso-CMS methods described herein. As such, the study showed that this genetic disease is easily, quickly, and non-invasively diagnosed using these methods.

Phenylketonuria (PKU) provides one example of a treatable IEM. PKU (OMIM 261600)) is an autosomal recessive IEM that results from a deficiency of a 452 amino acid enzyme (phenylalanine hydroxylase, PAH) encoded by 171 kB gene on chromosome 12q23.2 that mediates in phenylalanine (Phe) metabolism. The accumulation of toxic Phe metabolites causes growth failure, microcephaly, seizures, developmental delay, and severe intellectual impairment.

*Screening and treatment.* PKU is the first genetic disease to have an effective treatment, which is dietary restriction of ingested phenylalanine. Because of this, newborn screening for PKU is essential. Usually, blood from a newborn is obtained via a heel stick and collected on filter paper, and this is then analyzed via tandem MS. The upper reference limit for Phe in whole blood or plasma in neonates is <150 μmol/L (and is slightly lower (<120 μmol/L) in older children. Dietary Phe restriction is highly effective in preventing impaired neurological development, and should be continued at least throughout the period of neurodevelopment. Periodic monitoring of blood Phe levels is performed to assess dietary compliance.

The study sought to determine if the iso-CMS method could be used to diagnose PKU. Plasma samples were collected from 10 subjects, five were normal and five had PKU. Eight ul of plasma from each sample was place in 8 μL glass capillary tubes that were coated with EDTA (Vitrex Medical A/S, Denmark). The tube was placed in a centrifuge tube with 72 μL of a solution, which consisted of 25% isopropanol alcohol in water. The mixture was then vortexed thoroughly, and subsequent dilutions were prepared from this 1/10 dilution of the plasma sample. An aliquot of the diluted samples was mixed with an equal volume of 0.8 μM D8-phenylalanine, which was added as an internal standard. Three volumes of ice-cold acetonitrile were added, and proteins were precipitated and the mixture was centrifuged. The supernatant was obtained; it was evaporated to dryness, and then re-suspended in HPLC water. The analysis was performed with a semi-targeted metabolomic method (STMM) that uses Dansyl derivatization coupled with LC/MS analysis, which we have successfully used to analyze metabolomic changes in an un-biased manner in other systems. Dansyl derivatization improves metabolite separation on reverse phase columns and increases detection sensitivity by 10 to 1000 fold. The samples were dansylated using our previously described method (Wu M, Zheng M, Zhang W, Suresh S, Schlecht U, Fitch WL, Aronova S, Baumann S, Davis R, St Onge R, Dill DL, Peltz G. 2012. Identification of drug targets by chemogenomic and metabolomic profiling in yeast. Pharmacogenet Genomics 22: 877-86), and the dansylated samples were analyzed on an Agilent QTOF 6520 (Agilent, Santa Clara, CA), that was equipped with an Infinity 1290 UPLC system. Phenylalanine concentrations were calculated relative to those of a standard curve using Agilent QTOF Quantitative Analysis software. For comparison, amino acid concentrations in each plasma sample were measured in a Clinical Laboratory Improvement Amendments (CLIA) certified lab (the Stanford Pathology Clinical Lab) using their previously described methods. (Le A, Ng A, Kwan T, Cusmano-Ozog K, Cowan TM. 2014. A rapid, sensitive method for quantitative analysis of underivatized amino acids by liquid chromatography-tandem mass spectrometry (LC-MS/MS). J Chromatogr B Analyt Technol Biomed Life Sci 944: 166-74). To assess the correlation between the dansylated CMS and the direct plasma measurements, a Pearson's correlation was calculated and the statistical significance of the observed correlation coefficient was evaluated using R (www.r-project.org). The data was also evaluated using Spearman's rho statistic.

As shown in **TABLE 1,** the values obtained from the iso-CMS analysis were highly concordant with those obtained by the CLIA certified laboratory. The Pearson's correlation coefficient that was calculated for comparing the data obtained by the two different methods was 0.997, which indicated that the results obtained by the two methods were very highly correlated. A Spearman's rho statistic calculated for this data was 0.963.

**TABLE 1.** Plasma phenylalanine concentration (μM) measurements in plasma obtained from five normals (#1-#5) and five PKU subjects (#6-#10). The phenylalanine concentration in each plasma sample was measured by two different methods: (i) the iso-CMS method with dansyl labeling using 8 μl of sample; and (ii) direct amino acid measurement by ion pairing LCMS using plasma obtained from ml samples.

**TABLE 1.** Plasma phenylalanine concentration (μM) measurements in plasma

| Subject | Iso-CMS | Clinical Lab |
|---|---|---|
| 1 | 48 | 46 |

(continued)

| Subject | Iso-CMS | Clinical Lab |
|---|---|---|
| 2 | 36 | 38 |
| 3 | 59 | 60 |
| 4 | 60 | 52 |
| 5 | 51 | 53 |
| 6 | 195 | 200 |
| 7 | 252 | 262 |
| 8 | 221 | 224 |
| 9 | 340 | 366 |
| 10 | 191 | 179 |

**Example 3: Biomarkers for genetic diseases measured by iso-CMS**

In this example, blood markers for a genetic disease were accurately measured by the iso-CMS methods described herein. As such, the study showed that this genetic disease is easily, quickly, and non-invasively diagnosed using these methods.

**Maple Syrup Urine Disease** (**MSUD**, MIM #248600) is an autosomal recessive inborn error of metabolism (**IEM**). The normal activity of the branched-chain $\alpha$-ketoacid dehydrogenase (**BCKAD**) complex is disrupted by a mutation, and this interferes with the catabolism of branched chain amino acids (BCAAs), which include valine, leucine, and isoleucine. BCAAs are elevated in plasma and urine of affected subjects. MSUD is characterized by neurological and developmental delay, encephalopathy, feeding problems and a maple syrup odor to the urine. If untreated, MSUD can lead to metabolic decompensation, irreversible neurologic damage and death. A recent analysis of MS/MS screening data for five IEMs indicated that their birth prevalence ranged from 0.5 to 1.06 per 100K live births in Western populations: MSUD 0.71; Homocystinuria 0.53; Glutaric aciduria 1.06; Isovaleric Acidemia 0.79; Long-chain 3-hydroxyacyl CoA dehydrogenase deficiency 0.65. Their prevalence was usually higher when worldwide populations were examined.

BCAA catabolism is essential for normal physiological function, and most BCAA catabolism occurs in skeletal muscle (not liver). In the brain, BCAA metabolism is critical for maintaining glutamate levels, which is a neurotransmitter that plays important roles in brain development and function. Also, leucine accumulation is highly neurotoxic. BKAD is composed of E1 $\alpha$, E1 $\beta$, E2 and E3 subunits. Homozygous or compound heterozygous mutations in these subunits, which BKAD activity cause MSUD. The classic forms of MSUD present in the neonatal period and are associated with <2% of BCKAD enzymatic activity. There are also intermediate forms of MSUD, which are associated with 30% of normal BCKAD enzyme activity; and they present with episodic symptoms that are usually associated with catabolic states.

*Screening and treatment.* BCAAs are present in protein rich diets, and are among the 9 amino acids that are essential for human life. MSUD treatment consists of dietary BCAA restriction and metabolic monitoring. If treatment is initiated early, the outcome is very good. Therefore, newborns are routinely screened for MSUD. A rapidly performed high throughput reverse phase LC/MS method for quantitation of branched chain amino acid levels in blood spots and plasma was developed in 2011. This method can separate and quantitate leucine, isoleucine and alloisoleucine. Over 2.2M babies were screened in California over a 4-year period; 17 MSUD cases were detected and 3 were missed. A retrospective analysis of 5 missed cases indicated that none met the conventional criteria (Leucine > 200 $\mu$M, Leucine/Alanine ratio >1.5), and they appeared to have had an intermittent form of MSUC. Two of the missed cases had elevated levels of an isoleucine metabolite (allo-isoleucine). Thus, in some cases, it can be important to have 2nd tier screening for allo-isoleucine.

To determine if MSUD could be diagnosed using the iso-CMS method with dansyl-labeling, we measured leucine, valine and isoleucine levels in the 10 samples described above. As shown in **TABLE 2,** the values obtained from the iso-CMS analysis were highly concordant with those obtained by the CLIA certified laboratory.

**TABLE 2.** The plasma concentration ($\mu$M of branched-chain amino acids (BCAA) was measured in five normals (#1-#5) and five PKU subjects (#6-#10). The BCAA concentrations in each plasma sample were measured by two different methods: (i) the iso-CMS method with dansyl labeling; and (ii) direct amino acid measurement by ion pairing LCMS. The Pearson's correlation coefficient and Spearman's rho statistic that was calculated for comparing the data obtained by the two different methods is shown below each amino acid. The results obtained by the two methods were very highly correlated.

TABLE 2. Plasma concentration (μM) of branched-chain amino acids (BCAA)

| | Valine | | Leucine | | Isoleucine | |
|---|---|---|---|---|---|---|
| Subject | Iso-CMS | Clinical Lab | Iso-CMS | Clinical Lab | Iso-CMS | Clinical Lab |
| 1 | 320 | 327 | 236 | 233 | 129 | 132 |
| 2 | 132 | 141 | 62 | 65 | 30 | 33 |
| 3 | 247 | 263 | 116 | 117 | 59 | 60 |
| 4 | 257 | 249 | 142 | 124 | 72 | 62 |
| 5 | 362 | 402 | 283 | 290 | 187 | 197 |
| 6 | 188 | 212 | 95 | 101 | 44 | 51 |
| 7 | 193 | 219 | 84 | 89 | 33 | 36 |
| 8 | 232 | 259 | 146 | 151 | 83 | 92 |
| 9 | 316 | 350 | 153 | 163 | 56 | 62 |
| 10 | 247 | 248 | 138 | 142 | 71 | 79 |
| Pearson's Cor | 0.981 | | 0.993 | | 0.994 | |
| Spearman's rho | 0.891 | | 0.988 | | 0.960 | |

**Example 4: Measurement of the plasma levels of other amino acids**

To determine if other IEM could be diagnosed using the iso-CMS method with dansyl-labeling, we measured arginine, methionine and tyrosine levels in the 10 samples described above. As shown in **TABLE 3,** the values obtained from the iso-CMS analysis were highly concordant with those obtained by the CLIA certified laboratory. These results indicate that any amino acid can be quantitatively analyzed by the iso-CMS method with dansyl (or with other STMM) labeling.

**TABLE 3.** The plasma concentration (μM) of 3 other amino acids was measured in five normals (#1-#5) and five PKU subjects (#6-#10). The concentrations in each plasma sample were measured by two different methods: (i) the iso-CMS method with dansyl labeling; and (ii) direct amino acid measurement by ion pairing LCMS. The Pearson's correlation coefficient and Spearman's rho statistic that was calculated for comparing the data obtained by the two different methods is shown below each amino acid. The results obtained by the two methods were very highly correlated.

TABLE 3. The plasma concentration (μM) of Arginine, Methionine, and Tyrosine

| | Arginine | | Methionine | | Tyrosine | |
|---|---|---|---|---|---|---|
| Subject | Iso-CMS | Clinical Lab | Iso-CMS | Clinical Lab | Iso-CMS | Clinical Lab |
| 1 | 87 | 46 | 18 | 17 | 53 | 54 |
| 2 | 77 | 57 | 17 | 17 | 34 | 42 |
| 3 | 99 | 71 | 24 | 24 | 40 | 43 |
| 4 | 121 | 105 | 20 | 17 | 80 | 71 |
| 5 | 81 | 59 | 14 | 15 | 35 | 40 |
| 6 | 84 | 78 | 17 | 18 | 53 | 62 |
| 7 | 72 | 59 | 12 | 13 | 66 | 82 |
| 8 | 130 | 110 | 30 | 31 | 56 | 68 |
| 9 | 117 | 109 | 20 | 21 | 92 | 112 |
| 10 | 136 | 119 | 27 | 25 | 78 | 85 |
| Pearson's Cor | 0.925 | | 0.964 | | 0.934 | |
| Spearman's rho | 0.821 | | 0.902 | | 0.939 | |

### Example 5: Opiate analysis

The tertiary amines in the opiates described above ensure that they can be efficiently detected after electrospray ionization. Very sensitive and specific LC/MS assays have been developed, and they are extensively used for opiate quantitation. These assays are adapted here as iCOAMs for heroin, morphine, codeine, oxycodone and its metabolites (noroxycodone, oxymorphone), hydrocodone and its metabolite (hydromorphone), methadone, buprenorphine and fentanyl. Standard curves to assess the detection sensitivity for 8 of these opiates (morphine, codeine, oxymorphone, oxycodone, noroxycodone, hydrocodone, hydromorphone and fentanyl) (3 are shown in **FIG**. **9**) were developed.

The whole blood from the 8$\mu$L CMS sample was diluted 10-fold into a stabilizing solution comprising isopropanol in water (25%/75% V/V) (stabilizing solution). This was accomplished by inserting the capillary into a microcentrifuge tube and vortexing the sample until the blood had dispersed into the stabilizing solution. This is the iso-CMS sample.

In these studies, deuterated analogs (Sigma) were used as internal standards; and 50 ul of each iso-CMS blood sample was spiked with the indicated concentrations of a mixture of these internal standards. Cold acetonitrile was added to precipitate the proteins; the samples were dried and re-suspended in 5% acetonitrile in 0.1% formic acid, and analyzed on an Agilent QTOF6520 coupled with UHPLC infinity 1290 with a Phenomenex (Torrance, CA) poreshell kinetex C18 (2.0x100) column. Mobile phase A was 0.1% formic acid, mobile phase B was acetonitrile, and the gradient was run from 5% to 50% B in 15 minutes. The lowest limit of quantitation (LoQ) for each opiate was determined from the calibration curve. When 1/10 of the volume of a standard iso-CMS sample was injected, the LoQ for each of the 8 drugs was determined to be <0.5 ng/ml (**FIG. 9**). The known minimally effective plasma analgesic concentrations (MEAC) (ng/ml) for these opioids are: fentanyl (0.6), hydromorphone (1.5), morphine (8), and methadone (60). These results demonstrate that the MEACs for these opioids are easily detectable by our iso-CMS method.

These assays measure the [opiate] in simultaneously obtained iso-CMS and venipuncture samples obtained from opiate-treated subjects at SUH. Heroin is not used as a therapeutic in the US, but all of the other agents are frequently used in SUH operating rooms.

### Example 6: Measurement of therapeutic drugs

In addition to opiates, iso-CMS could be used to measure the plasma concentration of many therapeutic drugs and their metabolites. This is of great interest to patient safety, since when drugs are present outside of their therapeutic window, or the range of doses that produces therapeutic response without causing significant adverse effect in patients, they can become useless or dangerous. When drugs are present outside their therapeutic window, they no longer exert a therapeutic effect. When drugs are present above their therapeutic window, they begin to exert effects which are toxic to the patient. This is true even for drugs which are typically seen by the public as "safe."

Of particular interest is warfarin, which is a vitamin K antagonist with anticoagulant properties, and is commonly prescribed as an oral medication for the prevention and treatment of venous thrombosis as well as the prevention and treatment of the thromboembolic complications associated with atrial fibrillation. Warfarin has also been used to prevent recurrent transient ischemic attacks and to reduce the risk of recurrent myocardial infarction.

Warfarin therapy has an extremely narrow therapeutic window, and is associated with many drug-drug and drug-food interactions. This means that patients on warfarin therapy are at constant risk of adverse reactions due to overdose, which include severe bleeding and can lead to death. Thus patients on warfarin therapy must be constantly monitored and must submit to frequent blood tests.

Venipuncture-based blood tests can be painful and inconvenient. The iso-CMS method can make blood tests less painful and faster, and can reduce the amount of blood taken from a patient for each test. Thus this method can improve patient care outcomes for those on warfarin therapy or other drug therapy.

The whole blood from the CMS sample can be diluted 10-fold into a stabilizing solution comprising isopropanol in water (25%/75% V/V) (stabilizing solution. This may be accomplished by inserting the capillary into a microcentrifuge tube and vortexing the sample until the blood had dispersed into the stabilizing solution. This is the prepared CMS sample. This prepared CMS sample may then be subjected to LCMS analysis to determine the amount of warfarin in the blood, and compared with a gold standard for measurement. In light of the results of the other drug measurement examples below, it is expected that the iso-CMS method could be used to obtain a sample from which it is possible to measure warfarin or any other drug in the blood or other bodily fluid.

### Example 7: Accuracy of analysis of samples obtained via iso-CMS

Four drugs, dexamethasone, methadone, gabapentin, and ondanstetron, were administered to different subjects. Blood was taken from each patient using both venipuncture and the iso-CMS method, and the amount of drug in the samples taken by the iso-CMS method was compared with the amount of drug in the samples taken by the venipuncture (gold-standard) method.

15 human subjects were orally administered at least one safe dose of dexamethasone. After administration of the dexamethasone, the subjects were subjected to a waiting period of at least 30 minutes but not longer than 24 hours to allow for distribution of the dexamethasone throughout the body.

7 additional human subjects were orally administered at least one safe dose of methadone. After administration of the methadone, the subjects were subjected to a waiting period of at least 1 hour but not longer than 30 hours to allow for distribution of the methadone throughout the body.

8 additional subjects were orally administered at least one safe dose of gabapentin. After administration of the gabapentin, the subjects were subjected to a waiting period of at least 4 hours but not longer than 24 hours to allow for distribution of the gabapentin throughout the body.

44 additional human subjects who had recently undergone surgery were orally administered at least one safe dose of ondansetron. After administration of the ondansetron, the subjects were subjected to a waiting period of at least 2 hours but no longer than 12 hours.

In each of the four groups described above, at least 1 mL of blood was taken via venipuncture and the plasma was separated from the blood and stored (venipuncture sample). At the same time, 8 $\mu$L of blood was taken into a capillary using one embodiment of the methods described herein, which the iso-CMS method, as detailed below (CMS sample).

The whole blood from the CMS sample was diluted 10-fold into a stabilizing solution comprising isopropanol in water (25%/75% V/V) (stabilizing solution) This was accomplished by inserting the capillary into a microcentrifuge tube and vortexing the sample until the blood had dispersed into the stabilizing solution. This is the prepared CMS sample.

Both the venipuncture samples and prepared CMS samples were subjected to LCMS analysis, using protocols which were specific for the detection for the drug expected in each sample. E.g., samples from patients which had been administered dexamethasone were subjected to an LCMS protocol which is able to accurately and precisely quantify dexamethasone in a sample.

A standard curve for each drug was also prepared and analyzed on the LCMS instrument, such that the amount of drug in each sample could be quantified and compared with other samples. Using the appropriate standard curve for each drug, the amount of drug in each sample was calculated. The data were plotted as the plasma drug quantity as determined using the venipuncture sample versus the plasma drug quantity as determined using the prepared CMS sample. In this way, the reliability of the iso-CMS method to accurately measure amount of each drug in a sample was visualized.

For each plot, a linear curve was fitted to the data, and an $R^2$ value was calculated. The $R^2$ value is a measure of the correlation of the measurements, and ranges from 0 to 1, where 0 represents no correlation, and 1 represents a perfect 1: 1 correlation, or identical measurements between each sampling method. Additionally, the Pearson correlation and Spearman rank correlation were calculated for some of these datasets. The Pearson correlation is a measure of the linear correlation between two variables, ranging from 0 to 1, where 1 represents a perfect correlation. The Spearman rank correlation is a non-parametric measure of rank correlation that assesses how well the relationship between two variables can be described using a monotonic function. In brief, it ranges from 0 to 1 as well, where 1 again represents a perfect correlation between two variables.

In **FIG. 10,** the dexamethasone concentrations as measured by LCMS analysis of the two sampling methods are depicted in the plot, where each dot represents one patient, the x axis represents the plasma dexamethasone concentration in ng/ml as measured in the sample obtained using the iso-CMS method, and the y axis represents the plasma dexamethasone concentration in ng/ml as measured in the sample obtained using the venipuncture method. The linear fit was calculated and is depicted as the dashed line. The Pearson and Spearman rank correlation values were 0.95 and 0.93, respectively, indicating good correlation, suggesting that iso-CMS is able to accurately measure dexamethasone.

In **FIG. 11,** the methadone concentrations as measured by LCMS analysis of the two sampling methods are depicted in the plot, where each dot represents one patient, the x axis represents the plasma methadone concentration in ng/ml as measured in the sample obtained using the venipuncture method, and the y axis represents the plasma methadone concentration in ng/ml as measured in the sample obtained using the iso-CMS method. The linear fit was calculated and is depicted as the dashed line. The $R^2$ value was calculated as 0.91, indicating good correlation, suggesting that iso-CMS is able to accurately measure methadone.

In **FIG. 12**, the gabapentin concentrations as measured by LCMS analysis of the two sampling methods are depicted in the plot, where each dot represents one patient the x axis represents the plasma gabapentin concentration in ng/ml as measured in the sample obtained using the venipuncture method, and the y axis represents the plasma gabapentin concentration in ng/ml as measured in the sample obtained using the iso-CMS method. The linear fit was calculated and is depicted as the dashed line The $R^2$ value was calculated as 0.95, indicating good correlation, suggesting that iso-CMS is able to accurately measure gabapentin.

In **FIG. 13A and FIG. 13B,** the ondansetron concentrations as measured by LCMS analysis of the two sampling methods are depicted in the plot, where each dot represents one patient the x axis represents the plasma ondansetron concentration in ng/ml as measured in the sample obtained using the venipuncture method, and the y axis represents the plasma ondansetron concentration in ng/ml as measured in the sample obtained using the iso-CMS method. **FIG. 13A** represents data from all patients, including a subject with an unusually high level of plasma ondansetron. **FIG. 13B** shows the data for the subjects with ondansetron concentrations ranging from 15 to 60 ng/ml. The linear fit was calculated and is depicted as the dashed line. The $R^2$ value was calculated as 0.999, indicating excellent correlation, suggesting that iso-CMS is able to accurately measure ondansetron.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

**Claims**

1.  A method of performing an assay, the method comprising:

    preparing a test solution by adding a biological fluid in the amount of 1-50µL that has been obtained through capillary microsampling (CMS) to a solution comprising water and a stabilizing solvent, wherein the stabilizing solvent comprises methanol, ethanol, isopropanol or a mixture thereof; and
    performing an assay on the test solution to detect the presence and/or concentration of an analyte in the biological fluid, wherein the assay comprises mass spectrometry, chromatography, electrophoresis, or enzyme-linked immunosorbent assay (ELISA).

2.  The method of claim 1, wherein the biological fluid is blood.

3.  The method of claim 2, wherein the biological fluid is neonatal blood.

4.  The method of any one of claims 1-3, wherein the analyte is a drug, nutrient, or metabolite.

5.  The method of claim 1, wherein the stabilizing solvent comprises isopropanol.

6.  The method of any one of claims 1-5, wherein the solution comprises 10-75% V/V of the stabilizing solvent.

7.  The method of any one of claims 1-6, wherein the solution is substantially free of acetonitrile.

8.  The method of any one of claims 1-7, wherein approximately 2-25 µL, 4-15 µL, or 4-10 µL of the sample is obtained from the subject.

9.  The method of any one of claims 1-8, wherein the sample is diluted by the solution approximately 10-fold.

10.  The method of claim 1, further comprising cooling the sample.

11.  The method of any one claims 1-5, wherein the solution comprises 15-50%, 15-40%, or 20-30% V/V of the stabilizing solvent.

**Patentansprüche**

1.  Verfahren zum Durchführen eines Assays, wobei das Verfahren Folgendes umfasst:

    Herstellen einer Testlösung durch Zugeben eines biologischen Fluids in der Menge von 1-50 µl, das durch Kapillar-Microsampling (CMS) erhalten wurde, zu einer Lösung, die Wasser und ein stabilisierendes Lösungsmittel umfasst, wobei das stabilisierende Lösungsmittel Methanol, Ethanol, Isopropanol oder eine Mischung davon umfasst; und
    Durchführen eines Assays an der Testlösung, um das Vorhandensein und/oder die Konzentration eines Analyten in dem biologischen Fluid nachzuweisen, wobei der Assay Massenspektrometrie, Chromatografie, Elektrophorese oder einen enzymgebundenen Immunosorbens-Assay (ELISA) umfasst.

2.  Verfahren nach Anspruch 1, wobei das biologische Fluid Blut ist.

3.  Verfahren nach Anspruch 2, wobei das biologische Fluid Neugeborenenblut ist.

4.  Verfahren nach einem der Ansprüche 1-3, wobei der Analyt ein Arzneimittel, ein Nährstoff oder ein Metabolit ist.

5.  Verfahren nach Anspruch 1, wobei das stabilisierende Lösungsmittel Isopropanol umfasst.

6.  Verfahren nach einem der Ansprüche 1-5, wobei die Lösung zu 10-75 Vol.-% stabilisierendes Lösungsmittel umfasst.

7.  Verfahren nach einem der Ansprüche 1-6, wobei die Lösung im Wesentlichen frei von Acetonitril ist.

8.  Verfahren nach einem der Ansprüche 1-7, wobei etwa 2-25 $\mu$l, 4-15 $\mu$l oder 4-10$\mu$l der Probe von dem Subjekt erhalten werden.

9.  Verfahren nach einem der Ansprüche 1-8, wobei die Probe durch die Lösung etwa 10-fach verdünnt wird.

10. Verfahren nach Anspruch 1, ferner umfassend Kühlen der Probe.

11. Verfahren nach einem der Ansprüche 1-5, wobei die Lösung zu 15-50 Vol.-%, 15-40 Vol.-% oder 20-30 Vol.-% stabilisierendes Lösungsmittel umfasst.

**Revendications**

1.  Procédé de réalisation d'un essai, le procédé comprenant :

    la préparation d'une solution de test en ajoutant un fluide biologique d'une quantité de 1 à 50 $\mu$l qui a été obtenue par micro-échantillonnage capillaire (CMS) à une solution comprenant de l'eau et un solvant stabilisant, dans lequel le solvant stabilisant comprend du méthanol, de l'éthanol, de l'isopropanol ou un mélange de ceux-ci ; et
    la réalisation d'un essai sur la solution de test pour détecter la présence et/ou la concentration d'un analyte dans le fluide biologique, dans lequel l'essai comprend la spectrométrie de masse, la chromatographie, l'électrophorèse ou l'essai immuno-enzymatique (ELISA).

2.  Procédé selon la revendication 1, dans lequel le fluide biologique est du sang.

3.  Procédé selon la revendication 2, dans lequel le fluide biologique est du sang néonatal.

4.  Procédé selon l'une quelconque des revendications 1 à 3, dans lequel l'analyte est un médicament, un nutriment ou un métabolite.

5.  Procédé selon la revendication 1, dans lequel le solvant stabilisant comprend de l'isopropanol.

6.  Procédé selon l'une quelconque des revendications 1 à 5, dans lequel la solution comprend 10 à 75 % en V/V du solvant stabilisant.

7.  Procédé selon l'une quelconque des revendications 1 à 6, dans lequel la solution est sensiblement exempte d'acétonitrile.

8.  Procédé selon l'une quelconque des revendications 1 à 7, dans lequel approximativement 2 à 25 $\mu$l, 4 à 15 $\mu$l ou 4 à 10 $\mu$l de l'échantillon sont obtenus auprès du sujet.

9.  Procédé selon l'une quelconque des revendications 1 à 8, dans lequel l'échantillon est dilué par la solution environ 10 fois.

10. Procédé selon la revendication 1, comprenant en outre le refroidissement de l'échantillon.

11. Procédé selon l'une quelconque des revendications 1 à 5, dans lequel la solution comprend 15 à 50 %, 15 à 40 % ou 20 à 30 % en V/V du solvant stabilisant.

FIG. 1

FIG. 2A

FIG. 2B

FIG. 3A

FIG. 3B

FIG. 4A

FIG. 4B

FIG. 5

FIG. 6

FIG. 7A

FIG. 7B

FIG. 8

FIG. 9

FIG. 10

FIG. 11

FIG. 12

FIG. 13A

FIG. 13B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62580277

- US 62615576

**Non-patent literature cited in the description**

- **WU M ; ZHENG M ; ZHANG W ; SURESH S ; SCHLECHT U ; FITCH WL ; ARONOVA S ; BAUMANN S ; DAVIS R ; ST ONGE R.** Identification of drug targets by chemogenomic and metabolomic profiling in yeast. *Pharmacogenet Genomics,* 2012, vol. 22, 877-86

- **LE A ; NG A ; KWAN T ; CUSMANO-OZOG K ; COWAN TM.** A rapid, sensitive method for quantitative analysis of underivatized amino acids by liquid chromatography-tandem mass spectrometry (LC-MS/MS). *J Chromatogr B Analyt Technol Biomed Life Sci,* 2014, vol. 944, 166-74